# EUROPEAN PATENT APPLICATION

(11) **EP 4 177 352 A1**
(43) Date of publication of application: **10.05.2023**
(21) Application number: 21206745.8
(22) Date of filing: 05.11.2021
(51) Int. Cl.: C12Q 1/6806, C12Q 1/686

(54) **A MICROSPHERE COMPRISING A FIRST MATERIAL AND A SECOND MATERIAL**

(71) Applicant: Blink AG, 07747 Jena (DE)
(72) Inventor: ERMANTRAUT, Eugen, 07749 Jena (DE); ELLINGER, Thomas, 07745 Jena (DE); LEMUTH, Oliver, 07743 Jena (DE); STEINMETZER, Katrin, 07743 Jena (DE); KLINGNER, Susanne, 07743 Jena (DE); KANITZ, Lea, 07743 Jena (DE)
(74) Representative: Engelhard, Markus

(57) **Abstract**

The present invention relates to microsphere comprising a first material, preferably a first polymer, and a second material, preferably a second polymer. The present invention also relates to a liquid phase comprising a plurality of such microspheres. Furthermore, the present invention relates to a method for capturing a nucleic acid from a sample and to a method of amplifying a nucleic acid from a sample. The present invention also relates to the use of a plurality of microspheres in an assay for the detection of multiple nucleic acid analytes. Furthermore, the present invention relates to the use of a plurality of microspheres in an assay for the detection of a single nucleic acid analyte in multiple samples.

## Description

The present invention relates to microsphere comprising a first material, preferably a first polymer, and a second material preferably a second polymer. The present invention also relates to a liquid phase comprising a plurality of such microspheres. Furthermore, the present invention relates to a method for capturing a nucleic acid from a sample and to a method of amplifying a nucleic acid from a sample. The present invention also relates to the use of a plurality of microspheres in an assay for the detection of multiple nucleic acid analytes. Furthermore, the present invention relates to the use of a plurality of microspheres in an assay for the detection of a single nucleic acid analyte in multiple samples.

Chitosan has been used as a polymer for the capture of DNA in an aqueous system (Cao et al. 2006, Anal. Chem., vol. 78. pp. 7222-7228). A microchip with channels coated with Chitosan polymer was used to establish a totally aqueous purification protocol for DNA. Similar protocols have been also used successfully for RNA (Hagan et al. 2009, Anal. Chem., vol. 81, pp. 5249-5256; and Zhu et al., 2020, Micromaschines, vol. 11, p. 186, doi: 10.3390/mi11020186). Chitosan coated nylon membranes have been shown to be suitable matrices for enriching DNA from highly diluted solutions and for subsequent in-situ PCR on the membrane (Schlappi et al., 2016, Anal. Chem., vol. 88, pp. 7647-7653). Microspheres comprising agarose and non-cross-linked gelatine have been described in International patent applications WO 2021/122563 and WO 2021/122579.

There exists a need in the art for improved nanoreactor beads allowing an efficient capturing and/or amplifying of nucleic acids. There is also a need in the art for improved beads that are easier to handle and that are particularly suitable for use in capture and/or amplification protocols. There is furthermore a need in the art for providing improved beads that are versatile to be used, for example in multiplex assays involving multiple analytes and/or multiple samples. There is furthermore a need in the art for providing improved beads that are easier to locate and/or identify individually and/or to ascertain whether or not they have fused with other beads, in particular during a digital amplification and/or detection process. There is furthermore a need in the art for providing improved beads that allow identification and/or detection of a plurality of different beads via different detection channels.

In a first aspect, the present invention relates to a microsphere comprising a first material and a second material, wherein the first material is capable of forming a porous hydrogel when exposed to or comprising an aqueous solution, and has a melting temperature in the range of from 40°c to 90°C; and wherein the second material forms a network within said porous hydrogel, or wherein said second material is attached to said first material and both materials together form a network within said porous hydrogel; wherein said second material has a pKa-value and is capable of precipitating in an aqueous environment at a pH ≥ said pKa-value; wherein said network is capable of contracting when exposed to a temperature ≥ said melting temperature of said first material; said microsphere further comprising at least one of a) a fluorescent label and b) magnetic particles.

In one embodiment, said first material is a first polymer, and wherein said second material is a second polymer, an oligomer or a monomer; wherein
- if said second material is a second polymer, said second polymer is a polymer cross-linked with itself or is a polymer cross-linked with said first polymer;
- if said second material is an oligomer or a monomer, said oligomer or said monomer is attached to said first polymer.

In one embodiment, said second material, preferably said second polymer or said oligomer or said monomer, is furthermore capable of binding nucleic acids at a pH < said pKa-value, wherein, preferably, said pKa-value of said second material, preferably of said second polymer or of said oligomer or of said monomer, is > 6.

In one embodiment, said fluorescent label is selected from a) fluorescent particles having a size in the range of from 50 nm to 10 µm, preferably 100nm to 5 µm, more preferably 1 µm to 5 µm, and even more preferably 1 µm to 3 µm; b) non-particulate fluorescent dyes attached to said first and/or second material, preferably to said first and/or second polymer/said oligomer/said monomer; and c) combinations of a) and b).

In one embodiment, said magnetic particles have a size in the range of from 50 nm to 10 µm, preferably 100 nm to 5 µm, more preferably 1 µm to 5 µm, and even more preferably 1 µm to 3 µm.

In one embodiment, said first material, preferably said first polymer, comprises a binding member allowing the reversible and pH-independent immobilization of one or several amplification primers to said first material, preferably to said first polymer, wherein, preferably, for such reversible and pH-independent immobilization, said binding member specifically interacts with a binding entity attached to said one or several amplification primers; wherein, more preferably, said microsphere further comprises one or several amplification primers having a binding entity attached and being immobilized to said first material, preferably said first polymer, via said specific interaction between said binding member on said first material, preferably said first polymer, and said binding entity on said one or several amplification primers; wherein, even more preferably, said binding member is selected from avidin, streptavidin and derivatives thereof, and said binding entity is selected from desthiobiotin, iminobiotin, biotin having a cleavable spacer arm, selenobiotin, oxybiotin, homobiotin, norbiotin, diaminobiotin, biotin sulfoxide, biotin sulfone, epibiotin, 5-hydroxybiotin, 2-thiobiotin, azabiotin, carbobiotin, methylated derivatives of biotin, and ketone biotin; or vice versa. It should be noted that the binding member and the binding entity are chosen such that they interact with, i.e. bind to, each other in a reversible manner.

In one embodiment, said first material is a first polymer and is selected from agarose, gelatin, hyaluronan, elastin, elastin-like polypeptides, thermoresponsive polymers having an upper critical solution temperature (UCST), e.g. selected from poly(N-acryloyl glycinamide) (PNAGA), poly(allylamine)-co-poly(allylurea) and its derivatives, poly(methacrylamide), poly(N-acryloylaspargineamide), poly(N-methacryloylglutamineamide), poly(acrylamide)-co-(acrylonitrile). poly(sulfobetaine)s, poly(phosphorylcholine)s; and other polymers capable of forming a porous hydrogel when exposed to or comprising an aqueous solution, and having a melting temperature in the range of from 40°c to 90°C ; and said second material is either a second polymer selected from chitosan and derivatives thereof, gelatin and derivatives thereof, methylcellulose, poly(*N*-isopropylacrylamide) (pNIPAM), poly(ethyleneimine), poly(2-dimethyl(aminoethyl)methacrylate), poly(lysine), poly(histidine), poly(arginine) and polymer backbones having basic amino acids attached or incorporated as part of such backbone; or said second material is an oligomer, preferably selected from oligopeptides, said oligopeptides comprising or consisting of basic amino acid, such as histidine, lysine, and arginine; or said second material is a monomer selected from basic amino acids, such as histidine, lysine, and arginine;
with the proviso that when said first and said second material are a first and second polymer respectively, said polymers are different;
wherein, preferably, said first material is a first polymer that is non-cross-linked, and said second material is a second polymer that is cross-linked; or, equally preferably, said first material is a first polymer and said second material is a second polymer cross-linked with said first polymer; wherein, more preferably, said first polymer is agarose, and said second polymer is cross-linked chitosan.

In one embodiment, a microsphere additionally comprises an aqueous solution, such that said first material, preferably said first polymer is in the form of a porous hydrogel.

In one embodiment, a microsphere further comprises nucleic acid(s) which is(are) not an oligonucleotide primer or oligonucleotide primers, and which is(are)
- bound to said second material, preferably to said second polymer, if said aqueous solution has a pH< said pKa-value of said second material, preferably said second polymer, and
- still retained within the microsphere by or in said porous hydrogel of said first material, preferably said first polymer, if said aqueous solution has a pH ≥ said pKa-value of said second material, preferably said second polymer, but not necessarily bound, or not bound anymore, to said second material, preferably said second polymer.

Examples of such nucleic acid(s) which is(are) not an oligonucleotide primer or oligonucleotide primers and which may be bound to the second material or retained by the microsphere without being bound to the second material, are nucleic acids from a sample, such nucleic acids being nucleic acids of a microorganism or a virus, or, more generally, target nucleic acids the presence of which is intended to be tested or detected.

In one embodiment, said aqueous solution is either:
a) a first composition for washing and/or facilitating the binding of nucleic acids to said second material, e.g. said second polymer, said first composition having a pH-value < said pKa-value of said second material, e.g. said second polymer or, more preferably, said first composition comprising a buffer solution buffering in a pH-range < pKa-value of said second material, e.g. said second polymer; or
b) a second composition for facilitating and performing a nucleic acid amplification, said second composition comprising a buffer buffering in a pH-range suitable for performing a nucleic acid amplification, mono-nucleoside-triphosphates, an amplification enzyme, such as a suitable nucleic acid polymerase, e.g. Taq polymerase, optionally one or more amplification primers or one or more pairs of amplification primers if not already immobilized to said first material, preferably to said first polymer; and optionally a nucleic acid dye for the detection of an amplification product, such as an amplified nucleic acid; and/or, optionally, comprising respective molecular probes, e.g. TaqMan Probes, or molecular beacons; wherein said pH-range suitable for performing a nucleic acid amplification is > said pKa-value of said second material, e.g. said second polymer.

In one embodiment, said second material, preferably said second polymer is in a non-precipitated form. This may be particularly so when the respective microsphere has not been exposed to a pH-value ≥ pKa-value of said second material.

In one embodiment, the network within said porous hydrogel has not contracted. This may be particularly so when the respective microsphere has not undergone a heat treatment whereby it would have been heated to a temperature > melting temperature of the first material, preferably of the first polymer.

In one further embodiment, said second material, preferably said second polymer is in a non-precipitated form, and the network within said porous hydrogel of said microsphere has not contracted. This may be particularly so when the respective microsphere has not been exposed to a pH-value ≥ pKa-value of said second material, and when the respective microsphere has not undergone a heat treatment whereby it would have been heated to a temperature > melting temperature of the first material, preferably of the first polymer.

In another embodiment, said second material, preferably said second polymer is in a precipitated form. This may be particularly so when the respective microsphere has been exposed to a pH-value ≥ pKa-value of said second material.

In one embodiment, the network within said porous hydrogel has contracted. This may be particularly so when the respective microsphere has undergone a heat treatment whereby it would have been heated to a temperature > melting temperature of the first material, preferably of the first polymer.

In one further embodiment, said second material, preferably said second polymer, is in a precipitated form, and the network within said porous hydrogel of said microsphere has contracted. This may be particularly so when the respective microsphere has been exposed to a pH-value ≥ pKa-value of said second material, and when the respective microsphere has undergone a heat treatment whereby it would have been heated to a temperature > melting temperature of the first material, preferably of the first polymer.

In a further aspect, the present invention relates to a liquid phase that is immiscible with water and aqueous solutions, for example an oil phase, said liquid phase comprising a plurality of microspheres wherein the second material, preferably the second polymer, is in a non-precipitated form and/or wherein the network within said hydrogel has not contracted.

In a further aspect, the present invention relates to a liquid phase that is immiscible with water and aqueous solutions, for example an oil phase, said liquid phase comprising a plurality of microspheres wherein the second material, preferably the second polymer, is in a precipitated form and/or wherein the network within said hydrogel has contracted.

In a further aspect, the present invention relates to a method for capturing and/or enriching a nucleic acid or nucleic acids from a sample, said method comprising:
EITHER
a) Providing a plurality of microspheres as defined in any of claims 1 - 8, (i.e. a plurality of microspheres not (yet) comprising one (or several) nucleic acid(s) to be captured, enriched, amplified and/or detected, in a first composition for facilitating the binding of nucleic acids to said second material, preferably said second polymer, or exposing said plurality of microspheres to such first composition; said first composition having a pH-value < said pKa-value of said second material, e.g. said second polymer or, preferably, said first composition comprising a buffer solution buffering in a pH-range < pKa-value of said second material, e.g. said second polymer; thus allowing said plurality of microspheres to equilibrate with and incorporate said first composition; wherein preferably, said pKa-value of said second material, e.g. of said second polymer is > 6; and
b) Exposing said plurality of microspheres from step a) to a sample containing, or suspected of containing, nucleic acid(s), thus allowing said nucleic acid(s), if present, to bind to said second material, e.g. said second polymer, said sample, preferably, also comprising a buffer solution buffering in a pH-range < pKa-value of said second material, e.g. said second polymer, thereby capturing nucleic acid(s), if present in said sample;
   OR
a') exposing a plurality of microspheres, as defined in any of claims 1 - 8 to a sample containing, or suspected of containing, nucleic acid(s), thus allowing said nucleic acid(s), if present, to bind to said second material, e.g. said second polymer, said sample having a pH < pKa-value of said second material, e.g. said second polymer, wherein, preferably, said sample comprises a buffer solution buffering in a pH-range < pKa-value of said second material, e.g. said second polymer; thereby capturing nucleic acid(s), if present in said sample, as a result of such exposure.

In a further aspect, the present invention relates to a method of amplifying a nucleic acid from a sample, said method comprising performing the method for capturing and/or enriching a nucleic acid as defined herein in accordance with the present invention; and thereafter performing the step(s):
EITHER (OPTION A):
   c) exposing said plurality of microspheres from step b) or step a') to a second composition for facilitating and performing a nucleic acid amplification which comprises a second buffer solution buffering in a pH-range suitable for performing a nucleic acid amplification, mono-nucleoside-triphosphates, an amplification enzyme, such as a suitable nucleic acid polymerase, e.g. Taq polymerase, and, if said microspheres do not already comprise one or several amplification primers immobilized to said first material, preferably to said first polymer, via a specific interaction between a binding member on said first material, preferably said first polymer, and a binding entity on said one or several amplification primers, further comprising one or more amplification primers or one or more pairs of amplification primers; thus allowing said plurality of microspheres to equilibrate with and incorporate said second composition for facilitating and performing a nucleic acid amplification; said second composition, optionally, further comprising a nucleic acid dye for the detection of an amplification product, such as an amplified nucleic acid, and/or respective molecular probes, e.g. TaqMan Probes, or molecular beacons; wherein preferably, said pH-range suitable for performing a nucleic acid amplification is > said pKa-value of said second material, e.g. said second polymer;
   d) subj ecting said plurality of microspheres of step c) to an amplification protocol within said composition for facilitating and performing a nucleic acid amplification, such protocol involving or being preceded or followed by at least one step in which said suspension is heated to a temperature > melting temperature of said first material, preferably of said first polymer, such protocol, preferably, involving repeatedly and cyclically increasing and lowering the temperature to which said suspension of said plurality of microspheres is exposed, to levels allowing the amplification of nucleic acid(s), if present in said microspheres, that match the sequence(s) of said one or more amplification primers or one or more pairs of amplification primers;
OR (OPTION B):
   e) exposing said plurality of microspheres from step b) or step a') to a second composition for facilitating and performing a nucleic acid amplification which comprises a second buffer solution buffering in a pH-range suitable for performing a nucleic acid amplification, mono-nucleoside-triphosphates, an amplification enzyme, such as a suitable nucleic acid polymerase, e.g. Taq polymerase, and, if said microspheres do not already comprise one or several amplification primers immobilized to said first material, preferably to said first polymer, via a specific interaction between a binding member on said first material, preferably said first polymer, and a binding entity on said one or several amplification primers, further comprising one or more amplification primers or one or more pairs of amplification primers; thus allowing said plurality of microspheres to equilibrate with and incorporate said second composition for facilitating and performing a nucleic acid amplification; said second composition, optionally, further comprising a nucleic acid dye for the detection of an amplification product, such as an amplified nucleic acid, and/or respective molecular probes, e.g. TaqMan Probes, or molecular beacons; wherein preferably, said pH-range suitable for performing a nucleic acid amplification is > said pKa-value of said second material, e.g. said second polymer;
   f) transferring said plurality of microspheres from step e) to a liquid phase that is immiscible with water and aqueous solutions, for example an oil phase, thereby generating a suspension of said plurality of microspheres that are isolated from each other by said liquid water-immiscible phase, e.g. oil phase; and
   g) subjecting said plurality of microspheres of step f) to an amplification protocol, such protocol involving or being preceded or followed by at least one step in which said suspension is heated to a temperature > melting temperature of said first material, preferably said first polymer, such protocol, preferably, involving repeatedly and cyclically increasing and lowering the temperature to which said suspension of said plurality of microspheres is exposed, to levels allowing the amplification of nucleic acid(s), if present in said microspheres, that match the sequence(s) of said one or more amplification primers or one or more pairs of amplification primers, wherein, as a result of heating to a temperature > melting temperature of said first material, preferably said first polymer, the second material, preferably said second polymer, in said microspheres contracts, thereby generating a contracted core within each microsphere, wherein the fluorescent label and/or the magnetic particles, if present or comprised in the respective microsphere, accumulate within said contracted core, thus facilitating the detection and/or further manipulation of the respective microsphere, wherein, preferably, i) said fluorescent label and/or ii) said magnetic particles, accumulated within said contracted cores of said microspheres, are used to locate and/or identify individual microspheres or a plurality of microspheres.

In the above scheme of steps, OPTION A represents an embodiment of a method of amplifying nucleic acid(s) from a sample wherein the microspheres are not necessarily isolated from each other and mainly serve the purpose of capturing and enriching nucleic acid. Amplification is performed in a bulk volume surrounding the (plurality of) microspheres.

Option B represents an embodiment of a method of amplifying nucleic acid(s) from a sample wherein the microspheres are necessarily isolated from each other and each of them serves as an isolated reaction space/volume in which individual amplification reactions may occur. This embodiment is particularly useful for performing nucleic acid amplifications in digital or homogeneous format.

In one embodiment of the method for amplifying nucleic acid(s) from a sample, the method further comprises the step(s):
h) Detecting amplified nucleic acid(s), if present,
EITHER in said composition for facilitating and performing a nucleic acid amplification (OPTION A),
OR in said microspheres (OPTION B); wherein said amplification protocol results in the generation of an optically detectable signal which optically detectable signal is generated
EITHER in said composition for facilitating and performing a nucleic acid amplification (OPTION A),
OR in a space surrounding said contracted core of the respective microsphere (OPTION B);
and wherein detection of said optically detectable signal indicates the presence of amplified nucleic acid.

In one embodiment, individual microspheres or a plurality of microspheres are located and/or identified using i) said fluorescent label and/or ii) said magnetic particles, accumulated within said contracted cores of said microspheres.

In one embodiment, fused microspheres are distinguished from non-fused microspheres using i) said fluorescent label and/or ii) said magnetic particles, accumulated within said contracted cores of said microspheres; and/or iii) the presence of more than one contracted core within a fused microsphere; and/or iv) the presence of only one contracted core within a non-fused microsphere.

In a further aspect, the present invention relates to the use of a plurality of microspheres as defined herein in an assay for the detection of multiple nucleic acid analytes, said use comprising: performing the method for amplifying nucleic acid(s) from a sample according to the present invention as defined herein, and wherein said use involves different pluralities of microspheres, each plurality of microspheres comprising a different fluorescent label and being specific for the detection of a particular nucleic acid analyte by virtue of comprising a specific amplification primer for the amplification of said particular nucleic acid analyte or a specific pair of such amplification primers, wherein said different pluralities of microspheres can be detectably distinguished from one another by means of said fluorescent label, and wherein each plurality of microspheres is specific for a different nucleic acid analyte.

In a further aspect, the present invention relates to the use of a plurality of microspheres as defined in the present invention in an assay for the detection of a single nucleic acid analyte in multiple samples, said use comprising: performing the method for amplifying nucleic acid(s) from a sample according to the present invention as defined herein, and wherein said use involves different pluralities of microspheres, each plurality of microspheres comprising a different fluorescent label and further comprising an amplification primer for the amplification of said single nucleic acid analyte or a pair of such amplification primers, each plurality of microspheres being specific for a particular sample by virtue of being separately exposed in step b) or a') to such particular sample, wherein said different pluralities of microspheres can be detectably distinguished from one another by means of said fluorescent label, and wherein each plurality of microspheres is specific for the same nucleic acid analyte, and is specific for a different sample.

The present inventors have devised a new type of microspheres that are particularly suitable for nucleic acid capture and/or amplification protocols and that may be easily and individually identified and handled. The microspheres in accordance with embodiments of the present invention allow for an efficient binding/capture of nucleic acids from a sample by the respective microsphere and for a subsequent concentration/enrichment nucleic acids within the microsphere. The microspheres in accordance with embodiments of the present invention show a thermo-responsive behaviour which, for example, is particularly beneficial in protocols requiring identification and/or location and/or detection of individual microspheres. Without wishing to be bound by any theory, the present inventors believe that concentration/enrichment provides for an improved access/accessibility of any target nucleic acid to the amplification/detection reagents provided within the beads. Likewise, because the microspheres in accordance with the present invention also comprise at least one of a) a fluorescent label and b) magnetic particles, any such fluorescent label and/or magnetic particles will be concentrated within a small volume, namely in the interior of the microsphere within a small concentrated volume (herein also sometimes referred to as "core"), comprising the second, cross-linked material, which may be a (second) polymer, in precipitated form. The remainder of the volume provided by the microsphere is thus fully available for a detection/amplification reaction, whereas the fluorescent label and/or the magnetic particles are concentrated within a small volume and do not interfere with any such detection/amplification reaction. Moreover, because any fluorescent label and/or magnetic particles present within the microsphere are removed from the outer areas of the reaction volume defined by the microsphere, it is possible to read a target specific signal in the same fluorescence channel that is used for specific labelling of the microsphere (i. e. the code that is used to specify and designate the individual microsphere). This is because any target specific signal (i.e. signal from an amplified nucleic acid target) will be located in such outer areas of the reaction volume defined by the microsphere, meaning that the fluorescent label signal (=code) for the microsphere is physically and spatially separated from any target specific signal, thus allowing for a distinction between such two signals.

Microspheres in accordance with embodiments of the present invention are "hybrid" microspheres in the sense that they comprise a first material, preferably a first polymer, and a second material, preferably a second polymer, wherein the first material, preferably the first polymer, is non-cross-linked, and the second material, preferably the second polymer, is cross-linked. As used in this context, when the second material, e.g. second polymer is referred to as being cross-linked, this means that such second material, e.g. second polymer, is cross-linked to itself, or is cross-linked to said first material, e.g. said first polymer

In the following various statements are made with respect to exemplary embodiments involving a first polymer as "first material" and a second polymer as "second material". Such statements do however equally apply to embodiments, where the second material is an oligomer or a monomer that is attached to said first material, e.g. said first polymer.

The term "being attached", as used herein, typically refers to a linkage of one entity to another involving at least one covalent bond between such entities.

In preferred embodiments, the term "cross-linked" refers to a quality of the second material, e.g. second polymer, which is "cross-linked" in the sense that there is/are one or several covalent bonds that join or link together the second polymer to itself or to the first material, e.g. first polymer In other words, such "chemical cross-linking" refers to the intermolecular joining of two or more molecules of said second polymer (type) by one or several covalent bonds, or to the intramolecular joining of a single molecule of said second polymer (type) by one or more covalent bonds to itself, that is, different stretches of the chain of such molecule of said second polymer (type) are joined by one or more covalent bonds to each other, or to intermolecular joining of one or more molecules of the second polymer (type) to one or more molecules of said first polymer (type) by one or several covalent bonds.

In embodiments where the second material is an oligomer or a monomer, such oligomer or monomer is attached to said first polymer.

When microspheres in accordance with embodiments of the present invention are used in a protocol wherein the microspheres are contained in a liquid water-immiscible phase, such as an oil phase, and wherein such protocol involves or is preceded or followed by at least one step in which the microspheres are heated to a temperature > melting temperature of the first polymer, such first polymer undergoes a phase transition from the gel to the soluble state, and the reaction compartment provided for by each microsphere can be considered a droplet. Because, occasionally droplets fuse, this represents a potential problem in the handling, manipulation and/or processing, because different microspheres, i. e. droplets, maybe specific for different target analytes, as a result of which, different capture/amplification reactions may get mixed as a result of such fusion. However, because the microspheres in accordance with embodiments of the present invention comprise a second polymer which is cross-linked (or an oligomer or monomer that is attached to said first polymer) and which as a result of exposure of the microsphere(s) to a temperature > the melting temperature of the first polymer, forms a contracted network, such network of the second polymer becomes a compact structure that can be easily recognized within the volume of a microsphere (similar to an egg yolk within an egg). Such compact structure formed by the contracted network is herein also sometimes referred to as a "core" of a microsphere. Moreover, the contracted network of said second polymer (the "core") has also concentrated any fluorescent label and/or magnetic particles present within the microsphere, and therefore such contracted network provides a robust means to identify and/or locate and/or detect individual microspheres. It also provides an excellent means to control the fusing of microspheres and to identify any fused microspheres. This is, because such fused microspheres will contain as many contracted networks as microspheres form part of such fusion. These contracted networks (= "cores") can still be individually distinguished within an alleged single microsphere volume, and therefore such fused microspheres may be easily distinguished from non-fused microspheres.

As used herein, the term "microsphere", refers to spherical bodies, or substantially spherical or ellipsoidal or otherwise round-shaped, e.g. egg-shaped bodies, comprising a first material, preferably a first polymer, and a second material, preferably a second polymer,, as defined herein, having an average diameter in the range of from 20 µm to 500 µm, preferably from 20 µm to 200 µm, more preferably from 20 µm to 100 µm.

In accordance with embodiments of the present invention, the present inventors have provided a microsphere (and a plurality of such microspheres), comprising a first material, preferably a first polymer, and a second material, preferably a second polymer, wherein the first polymer, preferably, is a non-cross-linked polymer capable of forming a porous hydrogel when exposed to or comprising an aqueous solution, wherein the first polymer has a melting temperature in the range of from 40°C to 90°C; and wherein the second polymer is a cross-linked polymer having a pKa-value and is capable of precipitating in an aqueous environment at a pH ≥ said pKa-value; wherein the second polymer forms a network, wherein said network is capable of contracting when exposed to a temperature ≥ said melting temperature of said first polymer; and wherein said microsphere further comprises at least one of a) a fluorescent label and b) magnetic particles. In a preferred embodiment, said microsphere comprises both a) a fluorescent label and b) magnetic particles.

In preferred embodiments, the term "cross-linked" refers to a quality of the second polymer which is "cross-linked" in the sense that there is/are one or several covalent bonds that join or link together the second polymer to itself, or to the first material, e.g. first polymer. In other words, such "chemical cross-linking" refers to the intermolecular joining of two or more molecules of said second polymer (type) by one or several covalent bonds, or to the intramolecular joining of a single molecule of said second polymer (type) by one or more covalent bonds to itself, that is, different stretches of the chain of such molecule of said second polymer (type) are joined by one or more covalent bonds to each other, or to intermolecular joining of one or more molecules of the second polymer (type) to one or more molecules of said first polymer (type) by one or several covalent bonds.

When herein mention is made of the first polymer being preferably "non-cross-linked", this is meant to mean that such first polymer, as such, is not cross-linked with itself. Such non-cross-linked first polymer may nevertheless be part of a network wherein the second polymer is cross-linked in the aforementioned sense.

In embodiments according to the present invention, the second material, preferably the second polymer, has a pKa-value and is capable of precipitating in an aqueous environment at a pH-value that is greater than or equal to its pKa-value. Moreover, because, preferably, the second polymer is a cross-linked polymer, it forms a network, and such network is capable of contracting when being exposed to a temperature that is greater than or equal to a melting temperature of the first polymer. As a result of exposure of a microsphere in accordance with the present invention to an elevated temperature, such elevated temperature being greater than or equal to the melting temperature of the first material, e.g. first polymer, the network formed by the second material, e.g. second polymer, or by the second and first material together, will contract and will form a core, i. e. a defined volume within the overall volume of the microsphere, such defined volume being a smaller volume in comparison to the overall volume of said microsphere. Without wishing to be bound by any theory, the present inventors believe that in the formed network, stretches and regions of said second material, e.g. second polymer, are intertwined with stretches and regions of said first material, e.g. first polymer, and will, upon contraction of the network, pull in and concentrate also such intertwined regions and stretches of said first material, e.g. first polymer, within the contracted network. Such contracted network will therefore form a dense concentrated core within the microsphere.

In accordance with embodiments of the present invention, when the first material is a first polymer, it is preferred that the first polymer is a non-cross-linked polymer, meaning that it is not cross-linked to itself, i.e. it is non-cross-linked in the sense that it does not have one or several covalent bonds that would join or link together intermolecularly or intramolecularly the first polymer to itself. If however, in such embodiment, the second material is also a polymer, namely a second polymer, that is cross-linked with said first polymer, this could mean that the first polymer also becomes effectively "cross-linked" by virtue of being linked to said second polymer. In any case, however, the term "non-cross-linked" when used in the context of a first polymer, is meant to signify a scenario wherein such first polymer is not cross-linked wit or to itself.

A preferred first polymer is agarose. A preferred second polymer is chitosan. The term "agarose", as used herein, is meant to also include derivatives of agarose, as long as such derivatives are non-cross-linked (in the aforementioned sense), capable of forming a porous hydrogel when exposed to or comprising an aqueous solution, and have a melting temperature in the range of from 40°C to 90°C. The term "chitosan", as used herein, is meant to refer to a linear polysaccharide composed of randomly distributed β-(1→4)-linked D-glucosamine (deacetylated unit) and N-acetyl-D-glucosamine (acetylated unit). When such "chitosan" is the second polymer in accordance with embodiments of the present invention, it is capable of precipitating in an aqueous environment at a pH ≥ its pKa-value, and it is, preferably, cross-linked, as defined above. The term "chitosan" is also meant to include acid adducts and/or acid salts thereof, such as chitosan HCl, chitosan glutamate, chitosan lactate, chitosan acetate, as well as carboxymethylated forms thereof, such as N-carboxymethyl chitosan.

Because a microsphere in accordance with embodiments of the present invention also further comprises at least one of a) fluorescent label and b) magnetic particles, and, preferably, comprises both a) fluorescent label and b) magnetic particles, upon contraction of the network formed by the second polymer or by the second polymer together with the first polymer, such fluorescent label(s) and/or such magnetic particles will also become concentrated within said contracted network (core). Without wishing to be bound by any theory, the present inventors believe that the contracted network has an average pore size that is so small that it will "pull in" the fluorescent label(s) and/or magnetic particles, due to their larger size. Also, without wishing to be bound by any theory, such contraction therefore is believed to have multiple effects: Firstly, such contraction provides for making available a volume within the remainder of the microsphere which volume is the location of any amplification and/or reaction and/or detection to take place. Secondly, such contraction provides for a concentration of any fluorescent label and/or magnetic particles within the contracted network, thus facilitating the detection (→ by means of said fluorescent label) or the mechanical handling (→ by means of said magnetic particles) of said microsphere(s).

The term "fluorescent label" as used herein is meant to refer to fluorescent particles having a size in the range of from 50 nm to 10 µm, preferably 100 nm to 5 µm, more preferably 1 µm to 5 µm, even more preferably 1 µm to 3 µm; and to non-particulate fluorescent dyes that are attached to said first and/or said second material, preferably attached to said first and/or said second polymer, more preferably to said second polymer. In either case, i. e. whether the fluorescent label is particulate or non-particulate, a contraction of said network induced by exposing the microsphere to a temperature ≥ said melting temperature of said first material, preferably said first polymer, will lead to a concentration of said fluorescent label and/or said magnetic particles within the core, namely the contracted network formed by said second polymer alone or in combination with said first polymer. Accordingly, in preferred embodiments involving fluorescent particles, such fluorescent particles have a size that is larger than the average mesh or pore size of the contracted network.

The term "magnetic particles" as used herein, is meant to refer to particles, showing magnetic behavior, thus allowing such particles to be attracted by a magnet. In one embodiment, such magnetic particles are ferromagnetic particles. In another embodiment, such magnetic particles are paramagnetic particles. In another embodiment, such magnetic particles are ferrimagnetic particles. In yet another embodiment, such magnetic particles are superparamagnetic particles. In a preferred embodiment, such magnetic particles are ferromagnetic or paramagnetic particles. The term "magnetic particles", as used herein , is meant to exclude diamagnetic particles. "Magnetic particles", as used herein, preferably, have a size and average diameter or an average long extension in one dimension, in the range of from 50 nm to 10 µm, preferably 100 nm to 5 µm, more preferably 1 µm to 5 µm, even more preferably 1 µm to 3 µm. Preferably, the size and average diameter or average long extension in one dimension of the magnetic particles is chosensuch that it is larger than the average mesh or pore size of the contracted network and such that a contraction of said network induced by exposing the microsphere to a temperature ≥ said melting temperature of said first material, preferably said first polymer, will lead to a concentration of said magnetic particles within the core, namely the contracted network formed by said second polymer alone or in combination with said first polymer.

From the foregoing it follows that the average pore size of the contracted network is < 50 nm, preferably <25 nm, more preferably < 15 nm, and even more preferably < 10 nm.

As used herein, the phrase "said microsphere comprising at least one of a) a fluorescent label and b) magnetic particles", is also meant to envisage or include a scenario wherein such microsphere comprises more than one (type of) fluorescent label and/or more than (one type of) magnetic particles. In one embodiment of a microparticle(s) according to the present invention, the microparticle comprises at least two different fluorescent labels.

The microspheres in accordance with embodiments of the present invention are herein also sometimes referred to synonymously as "beads" or as "nanoreactor beads". Without wishing to be bound by any theory, the microspheres in accordance with embodiments of the present invention have the capabilities of a) undergoing a phase transition from a gel state to a soluble state, because of the first polymer's being non-cross-linked and having a melting temperature in the range of from 40°C to 90°C; and b) performing an internal rearrangement to the extent that the second polymer and the network formed by it contracts upon and when being exposed to a temperature that is greater than or equal to the melting temperature of the first polymer, thus leading to an internal compartmentalization within the microsphere, with the contracted network forming a dense region or volume or "core" within the greater (and less dense) volume provided by the microsphere itself. Such internal compartmentalization in the form of such "core" is visible and can be detected, and such detection is facilitated by enrichment and/or concentration of fluorescent label and/or magnetic particles within said core. At the same time, and in particular in embodiments of the invention wherein a microsphere is located in a liquid water-immiscible phase, i.e. wherein a microsphere is isolated from other microspheres by such water-immiscible phase, such aforementioned internal compartmentalization also generates a space surrounding said core in which space an amplification reaction and subsequent detection can take place. The microspheres in accordance with embodiments of the present invention are herein also sometimes referred to synonymously as "nanoreactor beads" or "nanoreactor microspheres" because they provide and define a reaction volume in which binding and/or amplification of a target nucleic acid may take place, which nanoreactor beads or microspheres, at the same time can be easily handled and detected.

Because such reaction volume of the nanoreactor beads or nanoreactor microspheres is of a magnitude in the nanoliter range, they are sometimes also simply referred to as "nanoreactors. For example microspheres with a mean diameter of 500 micrometers, 100 micrometers and 20 micrometers respectively have a volume of 65 nanoliters, 0.5 nanoliters and 0.004 nanoliters, respectively.

In a preferred embodiment, the amplification primer(s), i.e. the analyte-specific reagent(s) is(are) reversibly and pH-independently immobilized on said first material, preferably on said first polymer. "reversible" or "reversibly immobilised", as used herein in the context of one entity being "reversibly immobilised" on another entity is preferably meant to refer to a type of attachment in which the attachment between the entities can be "undone", under suitable conditions, but also allows for the entities to become attached again to each other, under suitable conditions. The release of such reversible immobilisation typically occurs and can be achieved in a manner that leaves the entities as such, and in particular their structure and binding capability, unchanged and allows for repeated cycles of attachment (i.e. binding) and release of the two entities to and from each other. A typical example of such reversible attachment is the hybridization of two complementary strands of nucleic acid under suitable conditions, e.g. hybridization conditions, which hybridization can be reversed if the resultant double strand is exposed to a different set of conditions, e.g. an increase in temperature, resulting in a melting of the double strand into single strands again. Upon cooling, the two strands may reanneal again, thus demonstrating the reversible nature of the attachment involved. An example of attachment which is not "reversible" in the aforementioned sense is the binding between wildtype biotin and wildtype streptavidin which is one of the strongest non-covalent binding events known and which cannot be reversed unless one or both entities are structurally altered or, possibly, even destroyed, e.g. by denaturation.

The reversible nature of the immobilisation of the amplification primer(s) on the respective first material, e.g. first polymer, in accordance with embodiments of the invention facilitates customized manufacture of microparticles in accordance with the needs of a user and also enables the adaptation of the respective libraries of microparticles for a wide variety of different methods. Depending on the respective point-of-care environment and the needs thereof, individualized and/or customized libraries may thus be easily fabricated. Moreover a reversible attachment of the analyte-specific reagents makes it possible to detach such analyte-specific reagents under circumstances where this may be desired, e.g. when such analyte-specific reagents should be available (and freely diffusible) for subsequent reactions, e.g. in a method protocol requiring the free availability of (freely diffusible) analyte-specific reagents. Preferably, such reversible immobilisation occurs by virtue of the first material, e.g. first polymer, having a binding member that allows the reversible immobilisation of an amplification primer on the first material. In a preferred embodiment, such binding member is attached to the first material, e.g. first polymer of the microsphere. Such binding member is designed and intended to bind to a binding entity which, in turn, is conjugated to the amplification primer. In this preferred embodiment, there is thus an interaction between a binding member attached to the first material of the microsphere, on the one hand, and a binding entity to which the amplification primer is conjugated, on the other hand. In this preferred embodiment, the binding member and the binding entity are chosen such that they interact with, i.e. bind to, each other in a reversible manner. As an example, the member attached to the first material may be streptavidin or a derivative thereof, and the binding entity to which the amolification primer is conjugated may be biotin or a biotin derivative, such as desthiobiotin, or vice versa (i.e. reagent binding molecule being biotin or a biotin derivative, such as desthiobiotin, and the binding entity being streptavidin or a derivative thereof), as long as the interaction between the two entities is reversible and binding can be reversed again, for example by a change, e.g. increase in temperature. For example, a nucleic acid primer (or a pair of nucleic acid primers, and, optionally, a detection probe), may be easily conjugated to desthiobiotin or may even be commercially provided in such desthiobiotinylated form. On the other side, the first material may have a binding member attached attached which may be streptavidin. It is extremely simple and easy for an end-user, under ambient conditions, to select a pair of desthiobiotin-primers, specific for an analyte of interest, and reversibly attach/immobilise this on the first material of the microsphere having streptavidin attached. A binding event between a biotin derivative and streptavidin under ambient conditions is easy to achieve by simply exposing the microsphere(s) comprising a first material having streptavidin as a binding member, to a solution of such (desthio)biotin-derivative-labeled primers, under suitable conditions. The immobilization can then be easily reversed by e.g. simply increasing the temperature.

The microspheres in accordance with the present invention show a thermo-responsive behavior and, because of the presence of two different polymers, are "hybrid" microspheres. Because of that, they are also sometimes referred to as "hybrid thermo-responsive" microspheres or beads.
Figure 1 shows possible approaches for the synthesis of microspheres ("bead-synthesis") in accordance with embodiments of the present invention.
Figure 2 shows various examples for synthesis of microspheres ("beads") involving agarose as the first material/first polymer and chitosan as the second material/second polymer.

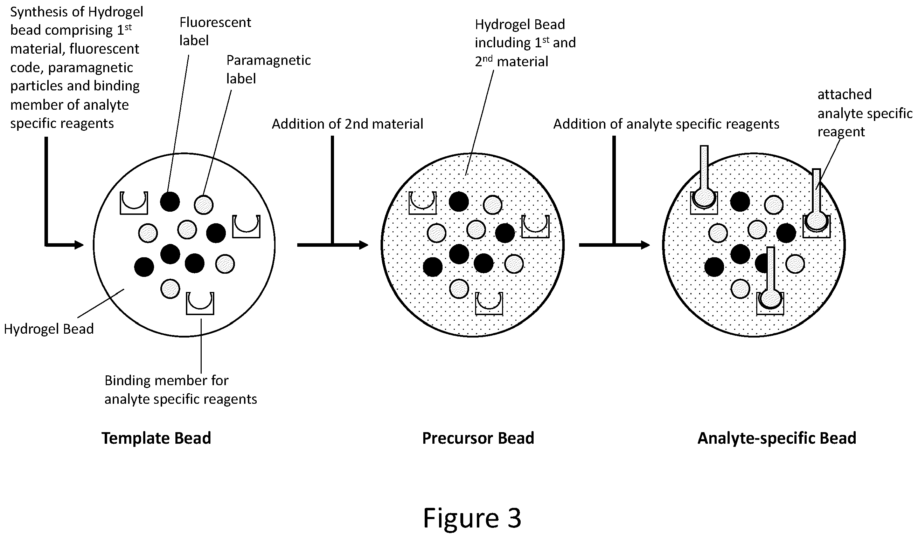

Figure 3 shows an embodiment involving microspheres ("beads") in which analyte specific reagents (e. g. amplification primers specific for a particular target) become attached/immobilized to the first material via a specific interaction between a binding member on the first material and a binding entity on the analyte specific reagent, with the analyte specific reagent having a binding entity attached that specifically interacts with the binding member on the first material. Also shown are the different stages, wherein the term "template bead" refers to a microsphere which only comprises a first material and not (yet) the second material which only becomes added in a further step, thus producing a "precursor bead". To such "precursor bead", then analyte specific reagents are added, thus converting the "precursor bead" into an "analyte-specific bead".
Figure 4 shows exemplary characteristic behavior of microspheres in accordance with embodiments of the present invention as produced in figure 3, depending on temperature and pH to which such microsphere (bead) is exposed.
Figure 5 shows a schematic view of a microsphere in accordance with embodiments of the present invention before and after it has been used in an amplification reaction.
Figure 6 shows a schematic workflow involving microspheres according to embodiments of the present invention (herein also referred to as "nanoreactor beads").
Figure 7 shows fluorescence image of microspheres with a fluorescence label attached to the second polymer in accordance with the present invention in the absence and presence of high concentration of a chaotropic salt. The beads show comparable fluorescence, which is indicative for the stability of the generated microsphere matrix.
Figures 8 shows photographs of 1% stained chitosan solution that has been mixed with buffers with an increasing pH from pH 2,0 to pH 9,0.
Figure 9 shows fluorescence images of microspheres incubated at pH 5,0 and pH 8,9 respectively and exposed to 25°C and 98°C, respectively.
Figure 10 shows embodiments of microspheres according to the present invention incubated at room temperature and 95°C and thereafter imaged at room temperature, wherein the microspheres contain carboxyfluorescein (FAM) as fluorescent label or cyanine-5 (Cy5) as fluorescent label, and these labels are detected in two different channels.
Figure 11 shows an embodiment where, based on the presence of fluorescent microparticles concentrated in their respective cores, the respective microspheres are assigned to different types of microspheres.
Figure 12 shows fluorescence signal intensities of outer microspheres compartments of the same microspheres shown in figure 11 indicating the amplification of molecular targets by PCR in some microspheres and lack of amplification in other microspheres
Figure 13 shows a standard calibration curve of gDNAcp output versus gDNAcp input (panel A) and figure 13, panel B shows images of a completed PCR protocol for the same chamber using two different fluorescence channels (green, lambda excitation 1 = 550 nm) (red channel, lambda excitation 2 = 635 nm). Whereby the green channels is used for recognizing all microspheres (segmentation, therefore all microspheres are labelled), and the red channel is used for detecting molecular targets by digital PCR (bright microspheres).
Figure 14 shows three images of a completed PCR protocol in a chamber, using three different fluorescence channels, namely one for label identification (red: lambda excitation 1 = 650 nm), one for detecting an HCV-specific amplification signal in some of the microspeheres indicated the number of targets in the analysed sample (blue: lambda excitation 2 = 470 nm) and one for applying an image segmentation algorithm for identifying the respective micrsopheres to be analysed (green: lambda excitation 3 = 550 nm).

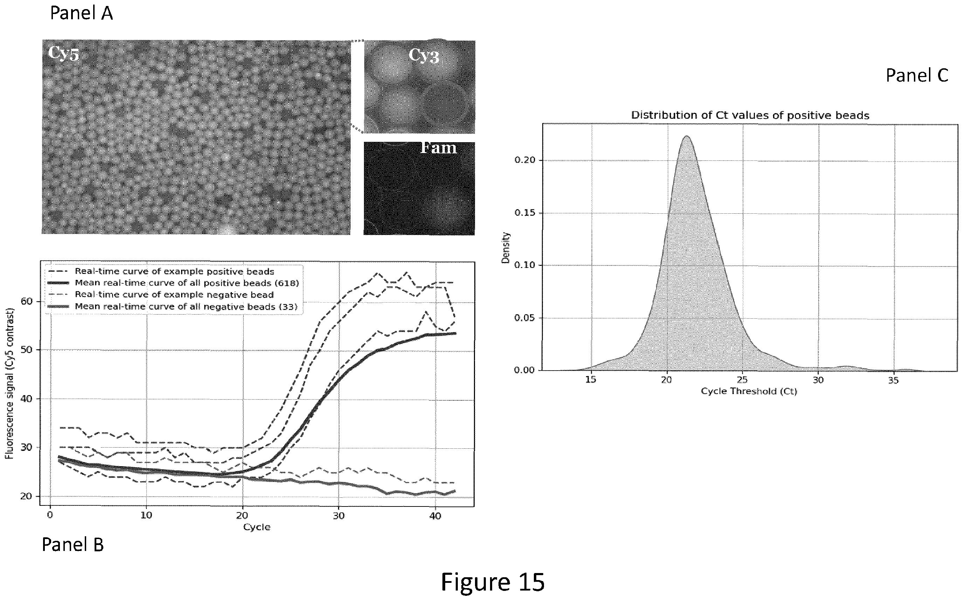

Figure 15 shows the monitoring of a microsphere digital PCR reaction with the detection of S. *aureus* genomic DNA; panel A shows the field of view for the Cy5 channel after amplification (PCR signal, bright and dark microspheres are clearly visible); Cy3 image taken before PCR reveals the analyte specific microspheres; the FAM image shows the control beads. Panel B shows the real time curves for Cy5 fluorescence for individual beads shown in the insets and the average intensity for the PCR positive and negative microspheres. Panel C shows the density distribution of Ct values estimated from all 618 valid beads of a positive microsphere type.

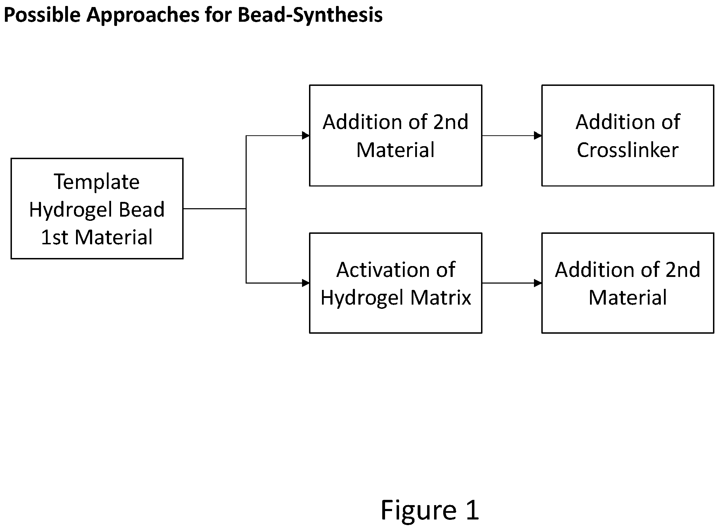

More specifically, and once more referring to figures 1-6, figure 1 shows possible approaches for ways of synthesis of microspheres (beads) in accordance with the present invention. In accordance with such figure 1, a hydrogel bead is produced using a first material, as described herein. The first material is capable of forming a porous hydrogel when exposed to or comprising an aqueous solution and has a melting temperature in the range of from 40°C to 90°C. Such a hydrogel bead comprising only a first material, but not the second material in accordance with the present invention, is herein also sometimes referred to as a "template hydrogel bead" or "template hydrogel microsphere". Starting from such template hydrogel bead, one alternative possibility of making a microsphere in accordance with the present invention involves the addition of a second material, in accordance with the present invention, and the subsequent addition of a cross-linker. The addition of such cross-linker, e. g. leads to a cross-linking of the second material to itself and/or to the first material.

Cross-linking chemistries are known to a person skilled in the art and typically involve primary amine-groups, carboxyl-groups, sulfhydryl-groups and/or carbonyl groups of the respective polypeptide chain(s). Suitable cross-linking agents are manifold and are for example summarized in the "Cross Linking Reagents Technical Handbook" by Thermofisher, downloadable under https://tools.thermofisher.com/content/sfs/brochures/1602163-crosslinking-reagents-handbook.pdf'. Examples of suitable crosslinking agents are bis-NHS-PEG (bis-N-succinimidyl-(pentaethylene glycol)ester) and glutaraldehyde. Other crosslinking agents involve carbodiimide, imido esters, maleimides, haloacetyl-groups, pyridyl disulfides, hydrazides, alkoxy amines, diazirines and/or aryl azides.

An example for this first alternative route of synthesis for microspheres in accordance with embodiments of the present invention is described in example 1 of the present application.

An alternative route for synthesis of microspheres in accordance with the present invention also starts from a template hydrogel microsphere comprising only a first material, which template hydrogel microsphere becomes activated. Activation, in this context is meant to refer to a process by which a suitable coupling reagent is added to the first material, thus making the first material more reactive to undergo a reaction with the second material. An example of such activation reaction is the activation of OH-groups of agarose as first material/first polymer by using 1,1'-carbonyl diimidazole (also abbreviated as CDI).

An example of this alternative route of synthesis for microspheres in accordance with embodiments of the present invention is described in example 2. This alternative route is also particularly suitable in cases where the second material is not a polymer, but is an oligomer or a monomer, as a result of which, such oligomer or monomer becomes attached to the first material.

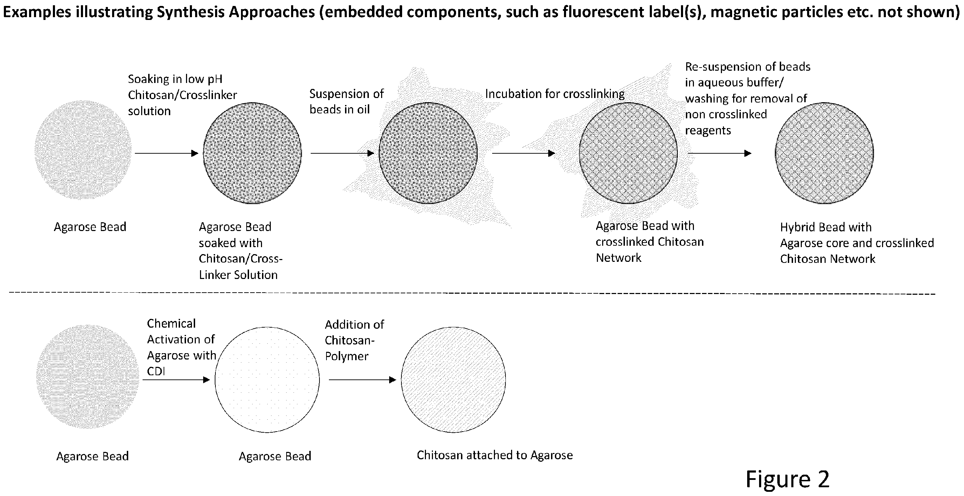

Figure 2 shows examples of the alternative synthesis routes of figure 1, using agarose as first material and chitosan as second material. The "crosslinking" alternative is shown at the top of the figure, whereas the "activation"-alternative is shown at the bottom of the figure.

Figure 3 shows a schematic diagram in which details are disclosed if the microspheres in accordance with embodiments of the present invention are to also comprise analyte specific reagents, for example amplification primers. The process starts with a so-called "template bead" or "template microsphere" which only comprises a first material in accordance with the present invention but already includes, in this example, fluorescent labels and (para)magnetic particles. Moreover, the first material comprises a binding member which allows the reversible and pH-independent immobilization of one or several amplification primers (= "analyte-specific reagent(s)"), wherein such binding member specifically interacts with a binding entity which itself is attached to said one or several amplification primers. Such reversible and pH-independent immobilization of one or several amplification primers may be achieved by appropriate choice of binding member on the first material and of the binding entity attached to the amplification primer(s), as explained further above. To such template-bead or template-microsphere, a second material, e. g. chitosan is added and soaked into such template bead, thus converting the "template bead" into a "precursor bead". The precursor bead, as opposed to the "template bead" now also includes the second material (e. g. chitosan) which may be cross-linked to itself and/or to the first material. Thereupon, the analyte specific reagents are added which become attached via the aforementioned specific interaction between the binding member on the first material and the binding entity on the analyte specific reagent(s) itself (themselves). The result of this process is a microsphere/bead which is specific for a particular analyte.

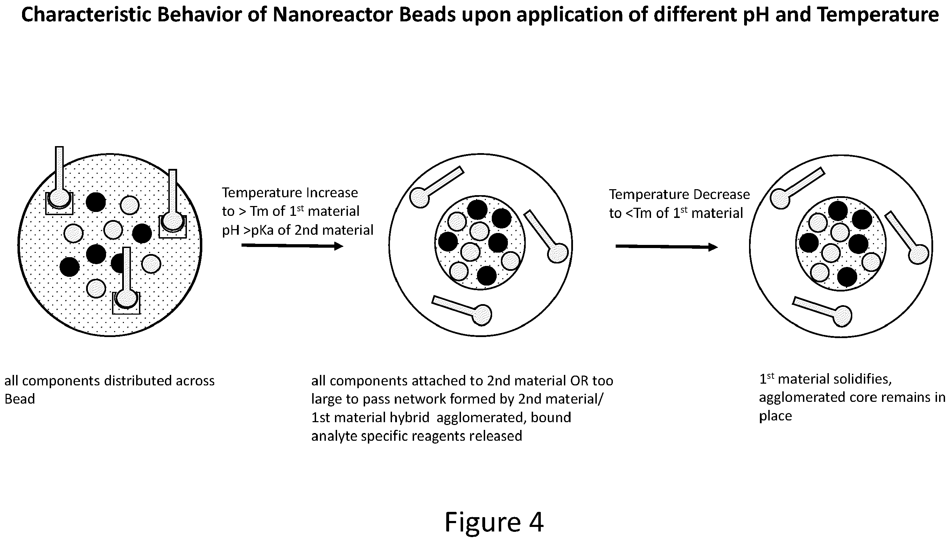

Figure 4 shows typical features of the analyte-specific microspheres resulting from the process described in figure 3, which are also referred to as "nanoreactor beads". It should, however, be noted that the presence of analyte specific reagents that would be reversibly attached to the first material/first polymer, is optional, in the sense that, for a subsequent amplification reaction, these analyte-specific reagents may also be separately added at a later stage, namely when an amplification reaction is to be performed. Reverting to figure 4, however, the embodiments shown therein do already have these analyte specific reagents which are reversibly attached, but in a pH-independent manner, and all components, including the first material and the second material (e. g. agarose and chitosan, respectively) are distributed throughout the microsphere. Upon a temperature increase to a temperature that is greater than the melting point of the first material, e. g. agarose, the network contracts and forms a contracted network involving the second material alone or the second material together with the first material. Upon an increase of the pH to a pH value that is > than the pKa value of the 2^{nd} material, the 2^{nd} material also precipitates, thus facilitating the contraction of the network. Because the pore size of the contracted network is smaller than the size of the fluorescent label(s) and/or of the (para)magnetic particles, these become concentrated/agglomerated within such contracted core. At the same time, however, because the temperature has increased above the melting temperature of the first material, the first material liquefies, and thus, there is or develops a space surrounding the contracted core in which space a solution is present, and such solution may contain, for example the analyte specific reagents which have become released because of the temperature increase. If additionally, the microsphere does or did also contain a nucleic acid of interest, such as a target nucleic acid, (such nucleic acid, however, not being shown in figure 4), such target nucleic acid would initially become enriched and/or bound to the second material as long as the pH would be < pKa of the second material, because of the second material's binding capability of nucleic acids at such pH. Once the pH was raised to a value > pKa value of the second material, the nucleic acid of interest would then also become released from its bound state to the second material and would become released into the space surrounding the contracted core.

In the second step shown in figure 4, the temperature is decreased again to a temperature smaller than the melting temperature of the first material, as a result of which the first material solidifies again whereas the agglomerated/contracted core remains in place and with it, the fluorescent label(s) and the magnetic particles located therein. The characteristic behavior of such microspheres in accordance with embodiments of the present invention, as shown in figure 4, applies in particular when such microsphere(s) has (have) been isolated within a liquid, water-immiscible phase, as a result of which such microsphere exists as a solid or semi solid particle, namely at temperatures below the melting point of the first material, or as a liquid droplet with a solid, contracted core, namely at temperatures above the melting temperature of the first material.

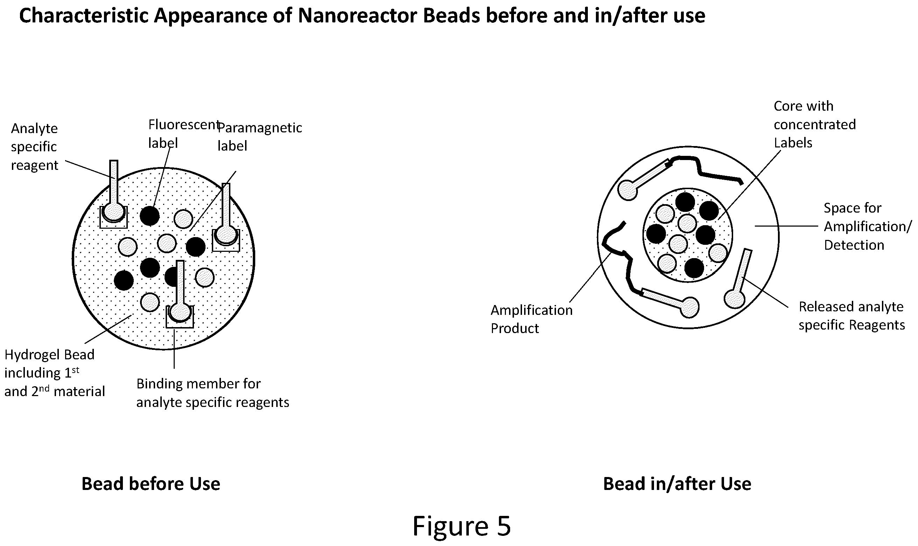

Figure 5 shows the characteristic appearance of one embodiment of microspheres in accordance with the present invention, for example the microspheres produced in figure 3 and shown in figure 4, i.e. involving analyte specific reagents, e. g. amplification primers. In such microsphere before use in an amplification reaction, the structure is as described for figure 4 and figure 3, i.e . all components are distributed throughout the microsphere, the analyte specific reagents are immobilized to the first material via a specific interaction between binding members on the first material and binding entities attached to the analyte specific reagents. During and after use, the microspheres are and/or have been exposed to a high-temperature step, involving a temperature that is higher than the melting point/temperature of the first material, as a result of which the second material has contracted and forms a contracted core, concentrating the fluorescent label(s) and magnetic particles within it. For such amplification reaction, the microsphere also becomes exposed to a sample containing or suspected of containing nucleic acid(s), in particular a specific target-nucleic acid. If such specific target-nucleic acid is present in such sample, it will become amplified during the amplification reaction in accordance with an amplification protocol, and the amplified nucleic acid will be located in the space surrounding the contracted core.

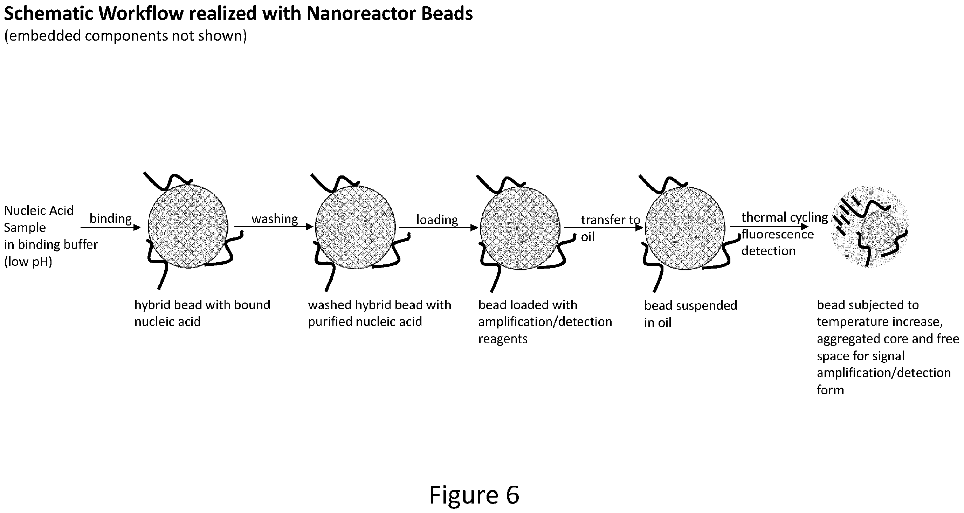

Figure 6 shows a schematic workflow that may be realized with microspheres in accordance with embodiments of the present invention of "nanoreactor beads". The bead/microsphere shown in this figure does not show the components also contained within such microsphere (shown on the previous figures), such as fluorescent label(s), magnetic particles, analyte specific reagents which are either attached to the first material initially or become added during performance of the amplification protocol instead, only the microsphere as such comprising a first material and a second material is shown in figure 6. Such microsphere is exposed to a sample containing or suspected of containing a nucleic acid in a buffer that has a pH which is smaller than the pKa of the second material. Because, in preferred embodiments, the pKa of the second material has a value > 6, the pH typically is a low pH-value in the acidic range as a result of such pH-value, the second material is protonated/ionized and thus positive, and therefore binds (any) (negatively charged) nucleic acid(s) contained in the sample. As a result, the microsphere has nucleic acid bound to it and within it, because the second material which has this affinity to nucleic acids and which is comprised in the microsphere is distributed throughout the microsphere. (Because such microsphere comprises both a first material and a second material, it is also sometimes referred to as a " hybrid" microsphere or "hybrid" bead in this figure.) The nucleic acid bound the microsphere is not shown. In a subsequent step, the microsphere becomes washed and is subsequently loaded with a composition for amplification. In preferred embodiments, the composition for washing is also a composition for facilitating the binding of nucleic acids to the second material and preferably, such composition for washing and/or facilitating has a pH-value which is still > the pKa-value of the second material.

In the "loading"-step, the microsphere is exposed to (e. g. imbibed with, soaked in, etc.) a further composition which is a composition for facilitating and performing a nucleic acid amplification reaction and detection. In preferred embodiments, such nucleic acid amplification reactions comprises a buffer buffering in a pH-range suitable for performing a nucleic acid amplification. Moreover, preferably, such composition also comprises mono-nucleoside-triphosphates, an amplification enzyme and, optionally, one or more amplification primers or one or more pairs of amplification primers if such primer(s) is (are) not already immobilized to the first material. Additionally, such composition for facilitating and performing a nucleic acid amplification and/or detection reaction may comprise a nucleic acid dye for the detection of an amplification product and, optionally may also comprise respective molecular probes, e. g. TaqMan, or molecular beacons. Preferably, and in many embodiments, the pH-value of such composition is > the pKa-value of the second material. Once the microsphere has been loaded with the composition for facilitating and performing a nucleic acid amplification, it is transferred into a liquid, water-immiscible phase, such as an oil phase, as a result of which, the microsphere becomes insulated, so to say, and exists as a droplet of an aqueous solution within a water-immiscible phase surrounding it. Once this has happened, the microsphere is subjected to an amplification protocol, involving or being preceded or followed by at least one step in which the microsphere (and the suspension comprising such microsphere(s)) is heated to a temperature > melting temperature of the first material. As a result of such heating step, the first material becomes liquefied, the second material contracts, and thus a phase separation within the microsphere occurs resulting in a contracted core surrounded by a space in which an amplification/detection reaction may or does take place. The fluorescent label(s) concentrated within the core allows an identification/detection of the microsphere. Likewise, the magnetic particles concentrated within the core allow an efficient handling/manipulation of such microsphere, and, depending on the presence or absence of other fluorescent dyes, for example attached to the first material, the presence or absence of molecular probes, numerous different channels of a detection scheme/instrument/system may be used for: locating and/or identifying individual microspheres; distinguishing fused microspheres from non-fused microspheres; distinguishing one microsphere from another microsphere; detecting and distinguishing different amplification products, if present; detecting and distinguishing the presence or absence of one or several amplification products in one or several samples to be analysed.

Microspheres in accordance with embodiments of the present invention are highly versatile in that there is no upper limit as to the number of different fluorescent labels used (as well as non-particulate fluorescent dyes used).

If, additionally, the microspheres then also contain non-particulate fluorescent dyes that are, for example, somehow associated with the first material then additional coding possibilities will arise, because then, not only the "colour" or, rather, mix of "colours", of the contracted core of the respective microsphere can be used for differentiation, but also the colour of the surrounding space (i. e. around such core).

Moreover, reference is made to the following examples, which are given to illustrate, not to limit the present invention:

### Examples

### Example 1

### Preparation of cross-linked Nanoreactor beads by linking molecules of second polymer to each other

### Preparation of agarose suspension with coding- and paramagnetic particles

Component 1: SeaKem ME agarose (Lonza)
Component 2: 1µm coding fluorescence particles (Thermo, λex=480nm, λem=520nm)
Component 3: 1µm Magnefy COOH magnetic particle (Bangslabs)

Component 1 ("first material", "first polymer") is weighed and dissolved in nuclease-free water (Roth) to preparate a 1% (w/v) homogenous solution. This is achieved by brief initial vortexing (20', 2000rpm) and heating with a thermomixer (Eppendorf) at 95°C for 10 minutes and 1200rpm. Unwanted impurities are removed with a 5µm Syringe-Filter (Whatman). To combine the agarose with the other components the temperature is then reduced to 80°C.

The preparation of the component 1 starts with 3 washes with the 10-fold volume in nuclease-free water (Roth). This step removes any undesired impurities in the storing buffer. To prevent agglomeration, the particles are ultrasonically treated at 23°C for 15minutes.

All components are stored in a polypropylene tube on a thermomixer (Eppendorf) at 80°C/1200rpm until further processing.

Typical final concentrations are :

| **Component** | **Typical final concentration** |
|---|---|
| Component 1 agarose | 0.5-5% |
| Component 2 coding particle | 2.5^{∗}10^{∧}5 - 2.5^{∗}10^{∧}6 particle/µl |
| Component 3 magnet particle | 2.5^{∗}10^{∧}5 - 2.5^{∗}10^{∧}6 particle/µl |

The agarose can be also modified with fluorescent dyes. Typical protocols are listed below.

### Staining of agarose with Cy3

### Materials used:

Agarose (A9793; SeaKem ME)
Cy3 Mono NHS Ester (GE)
50mM Potassium phosphate buffer, pH 8,3

5ml of a 1.5% agarose solution are prepared in 50mM potassium phosphate buffer, pH 8.3. For this, 75 mg of agarose is mixed with 5ml of buffer and incubated at 90°C for 20 min. After cooling to room temperature and solidification of the solution, the agarose is mechanically crushed and mixed with 1.6mg of dye solution dissolved in 50mM potassium phosphate buffer, pH 8.3. The final volume of the staining solution is 10ml. The incubation time for the staining is 120 min. During this time mixture is rotated at room temperature at 30 rpm on rotating wheel. Subsequently, the agarose is washed several times with distilled water until there is no free dye left in the supernatant. Finally, the agarose can be dried in the Speedvac and is ready for bead preparation.

### Generation ofmonodisverse template nanoreactor beads

Monodisperse template nanoreactor beads (="microspheres") are generated on a modified µEncapsulator system (Dolomite microfluidics) with the described components, agarose, chitosan, and coding- and magnetic particles in a one-step process of emulsion formation using a simple flow-focus device. In detail, a standard junction chip (100µm) of fluorophilic nature is used with a 4-way linear connector and a chip interface H to interface the fluidic connection between tubing and chip. Two Mitos P-Pumps deliver the agarose/particle suspension and the carrier oil with emulsifier, e.g. PicoSurf (Sphere Fluidics, 2.5-5% in Novec 7500, 3M).

The off-the shelf system is modified with a heating rig which is placed on top of a hot plate and allows for maintaining the agarose/chitosan/particle suspension in liquid state and heating up the driving fluid ensuring consistent temperature when carrier oil and the components suspension contact at the chip junction. The heating cover of the agarose/chitosan/particle suspension reservoir prevents unwanted evaporation. A magnetic stir bar is used to hold the suspension intact (300-600 rpm controlled by hotplate).

For bead production, the temperature of the heating rig is set to 75°C. The fluid lines are primed at 2000mbar for 1 min using the Flow Control Software. A flow rate of 10-50µl/min is adjusted for stable droplet formation. Parameters, like diameter, are monitored with the Dolomite Flow Control Advanced Software.

The monodispersed beads are collected through a 200µm PTFE-Tubing, connected to the outlet of the microfluidic chip. In the tubing the beads cool down and are finally collected in a tube (e.g. 15ml, BD), placed on ice to initiate solidification of the hybrid hydrogel. To prevent loss of the aqueous phase of the beads at the oil-air boundary, the sample is overlayed with 500-1000µl of nuclease-free water (Roth). The template nanoreactor beads are cooled down to 4°C for at least 1h to form stable hybrid polymer scaffolds.

### Recovery of template nanoreactor beads from the oil suspension

Solidified template nanoreactor beads accumulate on top of the emulsion oil in the collection tube. The Picosurf oil is removed carefully with a pipette taking care not to remove the beads. Residues of Picosurf are further diluted with the solvent Novec 7500 (3M, 1:1 volume of collected bead). Afterwards, 1H,1H,2H,2H-perfluorooctanol (PFO, Sigma, 1:4 volume to collected beads) is added to break the emulsion. Also, nuclease-free water (Roth, 3:1 volume of collected beads) is added. Now the Beads float to the water phase. To accelerate this process, the tube is vortexed for 5s and centrifuged at 2500rpm for 5s.

Finally, the beads are carefully harvested by a pipette from the water layer of the tube and transferred to a fresh tube. This procedure can be repeated to remove residual fluorocarbon oil and surfactant. Recovered beads are washed once with nuclease-free water (Roth, 3:1 volume of collected bead).

### Generation of cross-linked nanoreactor beads by Cross-linking of Chitosan

A 2% Chitosan solution is prepared and mixed with approximately a suspension containing approximately 10.000 of the previously generated template nanoreactor beads. Bis-N-succinimidyl-(pentaethylene glycol) ester (BIS-NHS-PEG; SIGMA-ALDRICH) is used as a cross-linker for the Chitosan. A linker solution is prepared by dissolving 1 mg of solid BIS-NHS-PEG 1 mL of distilled water, 250µL of this solution are added to the suspension. The suspension is vortexed. The vortexed suspension is allowed to settle for 30 seconds. 20 mL Picosurf oil (Sphere Fluidics) is added to the suspension, which is immediately put an a Bead Ruptor device (OMNI) and shaken at 4m/s for three times 5 seconds. Thereafter the Beads-in-Oil suspension is put to a refrigerator and kept there over night at 4°C.

The cross-linked template nanoreactor beads are recovered from the Picosurf oil according to the protocol described for the recovery of template nanoreactor beads.

If the chitosan itself is to be labelled by a non-particulate fluorescent dye, a typical protocol is listed herafter and may be performed prior to using such labelled chitosan in the above protocol for the generation of cross-linked nanoractor beads:

### Staining of chitosan with Cy5

### Materials used:

Chitosan HCl (Heppe Medical chitosan GmbH).
Cy5 Mono NHS Ester (GE)
Distilled water

1g of chitosan HCl is dissolved in distilled water and a 2% solution is prepared. 1.6 mg Cy5-Mono NHS Ester is dissolved in 100 µl distilled water and added to the chitosan solution. After short mixing, the solution is incubated overnight on a rotary wheel at room temperature and 30 rpm. Subsequently, the solution is dialyzed against distilled water for a total of 48 h, exchanging the water three times. The stained chitosan polymer is then concentrated to a 2% solution in a Speedvac.

### Example 2

### Preparation of cross-linked Nanoreactor beads by linking first polymer molecules with second polymer molecules

In the following embodiment, the generation of hybrid hydrogel beads consisting of an agarose scaffold and a capture matrix composed of a second material which is Chitosan polymer applied as the ionizable capture matrix of the nanoreactor beads is shown. In this example the Chitosan is cross-linkedto the Agarose matrix, whereby cross-linking is achieved by binding the Chitosan to chemically activated Agarose polymer comprising the matrix of premade hydrogel beads As a result the Chitosan molecules are bound to Agarose molecules which form linkages between different Chitosan molecules. In addition to the classical chitosan, various water-soluble derivatives are available and can be used interchangeably:
- Chitosan HCl
- Chitosan Glutamat
- Carboxymethylchitosan
- Chitosan Lactate
- Chitosan Acetate

### (Heppe Medical chitosan GmbH)

### Generation of monodisperse Agarose beads

Monodisperse beads composed of agarose are fabricated using a modified the µEncapsulator system (Dolomite microfluidics). The solidified agarose beads are collected from the emulsion oil. The excess emulsion oil is carefully removed with a pipette. After adding 1H,1H,2H,2H-perfluorooctanol and distilled water, the emulsion is broken and beads are subsequently transferred to the aqueous phase. After washing the agarose beads several times with distilled water, the beads are available for further treatment with chitosan.

### Reacting the activated Agarose beads with cross-linked chitosan

A vial containing 1,1'-carbonyldiimidazole (CDI; Sigma) as the coupling reagent is brought to ambient temperature before opening to prevent condensation. The reaction takes place in 2 steps:

### Step 1: Activation of OH groups of the agarose

The agarose bead suspension is centrifuged at 700rpm for 1min and the supernatant is discarded. CDI is dissolved in 2 mM HCl to give a 200mM solution. The beads are then mixed with the CDI solution in a 1:1 ratio so that the reaction takes place in 1mM HCl. The reaction tube is incubated for 10 min on a rotating wheel at 30rpm and room temperature.

After the reaction, the beads are washed by centrifuging at 700 rpm for 2 min. The supernatant is removed, and the collected beads are resuspended in an equal volume of distilled water and mixed. The washing procedure is repeated twice so that the activated beads are prepared for subsequent chitosan binding.

The activation reaction should be carried out at a pH <5.0. Alternatives to 1mM HCl for adjusting pH are for example:
- 20mM acetate buffer; pH 4.0
- 20mM citrate buffer; pH 4.0
- 5mM malonic acid; pH 3.0

### Step 2: Coupling of chitosan to activated agarose polymer comprising pre-made precursor beads

A 1% chitosan solution is prepared in distilled water. In the second step, the activated beads and the 1% chitosan solution are mixed immediately in a 1:1 ratio. The mixture is incubated for 30 min on the rotating wheel at 30 rpm and room temperature. After the reaction, the beads are washed by centrifuging at 700 rpm for 2 min. The supernatant is removed, and the beads are resuspended in an equal volume of distilled water and mixed. The washing procedure is repeated at least ten times so that there is no free chitosan in the supernatant.

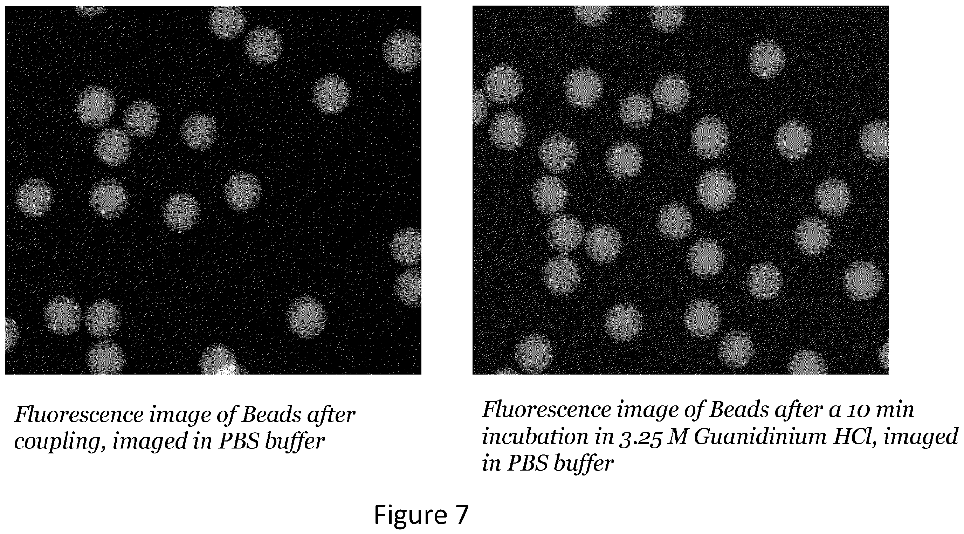

The stability of the cross-linked polymer beads is checked by using a chitosan stained with Cy5 and after adding a high concentration (3.25 M) of a chaotropic salt (e.g. Guanidinium HCl). The images of figure 7 show the stability of the achieved coupling under chaotropic conditions.

### Characterisation of the solubility of chitosan at different pH values

In this section, an attempt is made to explain the specific behavior of microspheres in accordance with the present invention.

### Used materials:

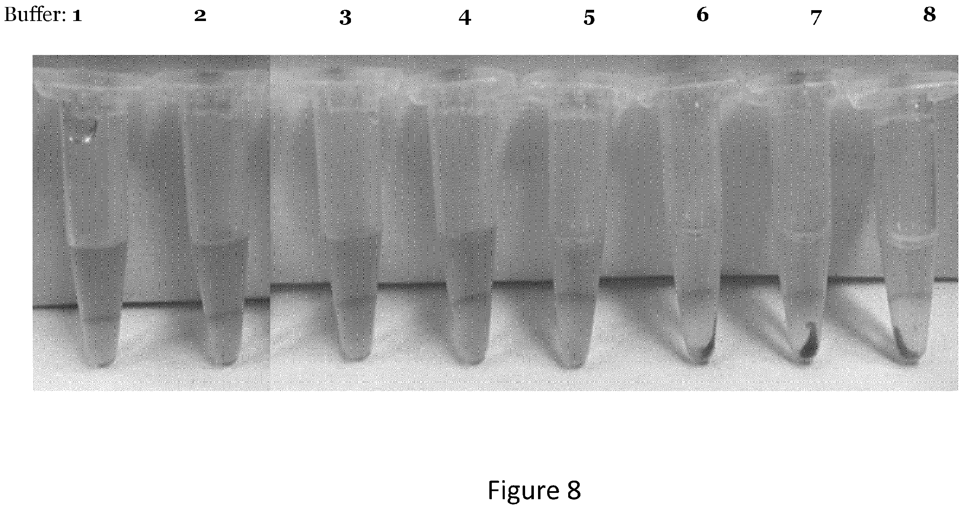

Chitosan-HCl (Heppe Medical chitosan GmbH), for better visibility a Cy5- coupled chitosan (see protocol above) is used
Malonic acid
MES (2-(N-morpholino)ethanesulfonic acid) buffer
Potassium phosphate buffer 250µl of a 1% stained chitosan solution is mixed with buffers with an increasing pH and incubated for 10 min at room temperature. The solutions are then centrifuged at 2000 rpm for 2 min. The result is shown in figure 8, wherein the used buffers are:
   **1** 50 mM Malonic acid; pH2,0
   **2** 50 mM Malonic acid; pH3,0
   **3** 50 mM Malonic acid; pH4,0
   **4** 50 mM MES buffer; pH5,0
   **5** 50 mM MES buffer; pH6,0
   **6** 50 mM MES buffer; pH7,0
   **7** 50 mM Potassium phosphate buffer; pH8,0
   **8** 50 mM Potassium phosphate buffer; pH9,0

The results show that at a pH below 6 the solution remains intact, whereas above the pKa of chitosan, at pH7 and above precipitation of the polymer occurs.

Without wishing to be bound by any theory, this pH dependent precipitation/agglomeration of the chitosan polymer is also observed with the hybrid beads and is believed to form an important part of the invention.

For the following experiment, two different buffers were used:
**Buffer 1:** 200mM MES buffer; pH5,0, 100mM KCl
**Buffer 2**: 200mM Tris/HCl; pH8,9, 100mM KCl

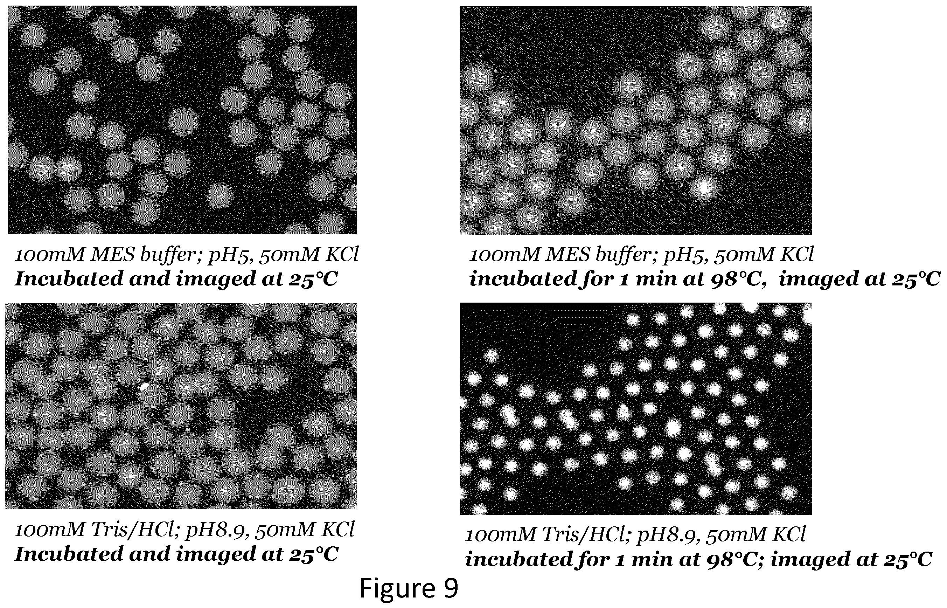

50µl beads as produced in this Example 2 are mixed with 50µl **buffer 1** and 50µl of the same beads are mixed with 50µl **buffer 2.** After 1 min incubation, the beads are sedimented by centrifugation at 700 rpm for 1 min. The supernatant is removed. The beads are then transferred to a non-aqueous phase (e.g. Picosurf Oil) and then transferred to a reaction and detection chamber (RDC), as described for example in Loncarevic IF, et al., PLOS ONE 16(3): e0242529. https://doi.org/10.1371/journal.pone.0242529 , and incubated at different temperatures. The results are shown in Figure 9.

Without wishing to be bound by any theory, whilst the present inventors have shown such behavior for cross-linked chitosan as a second polymer, they expect the same behavior also from other polymers as well if used in a cross-linked form as second polymer, e.g. Gelatin, poly(ethylenimine), poly(2-dimethyl(aminoethyl) methacrylate), poly(l-lysine), poly(histidine) and similar polymers. Moreover, it is possible to use Oligomers or monomers coupled to the activated first polymer. Such oligomers or monomers can be oligo- or mono-histidine, oligo- or mono-lysine, oligo- or mono-arginin and other chemically similar molecules. Likewise, the present inventors have shown such behavior for agarose as a first polymer, however, they expect the same behavior to be achieved by other polymers as well if used in a non-cross-linked form, for example by non-cross-linked gelatin, methylcellulose, hyaluronan, elastin-like polypeptides, other derived polymers with similar gel forming and thermal response characteristics. Also poly-N-isopropylacrylamide and its derivatives can be used for this purpose.

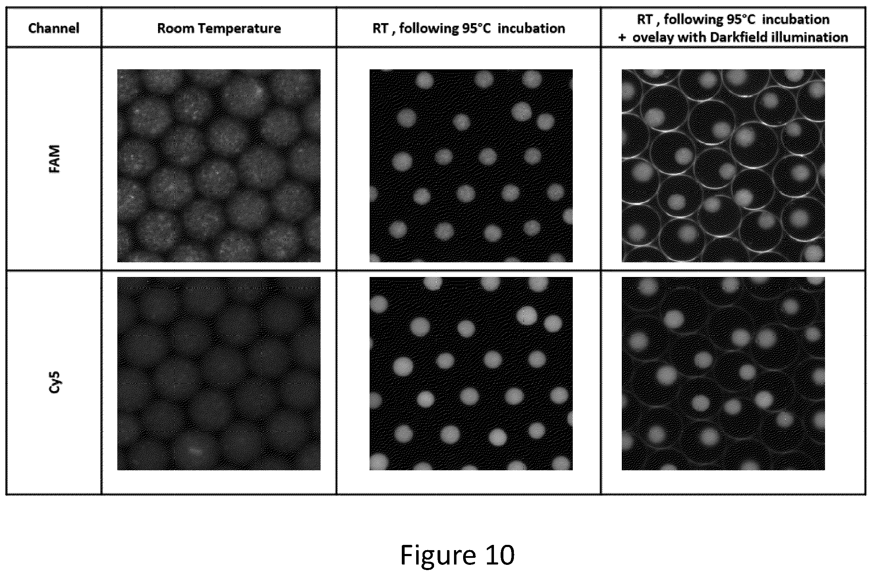

Beads generated according to the listed protocols and treated with appropriate buffers show a pre-determined response to a change in temperature. This is illustrated by figure 10.

The images of figure 10 clearly show an agglomeration of the fluorescent particle label following a temperature increase. Upon cooling to room temperature, the first polymer and thus the microsphere solidifies, however now with all the embedded microparticles concentrated within the core agglomerate formed by the contracted second polymer.

### E) Labelling/Coding of microspheres (beads) and amplification reactions using such labelled/coded microspheres (beads)

### Basics

Sample and analyte multiplexing requires that each bead type be uniquely encoded to distinguish one bead type from another. The strategy for bead encoding is based on either labelling the second polymer or the embedded microparticles with with fluorescence dyes for four separate fluorescence channels (blue, green, red and darkred). After heating all labelling/coding particles are located in the inner compartment and are clearly delineated from fluorescence signal in the outer compartment of nanoreactor beads.

For adjustment of fluorescence levels, a binary coding can be applied, meaning fluorescence dye in a specific channel is either present or not. The maximum number of bead types that can be encoded using this strategy is 2⁴ = 16, where two is the number of resulting fluorescence intensity levels (0 and 1) and four is the number of fluorescent channels. The number of possible bead types can be further increased by allowing multiple levels of fluorescence dye concentrations or by the extension to agarose level coding detectable in the outer compartment of beads, if this is not used for monitoring/detecting the enzymatic amplification reaction indicative for the presence of an analyte/target

### Image Analysis

The image analysis software utilizes a segmentation algorithm that identifies circular object contours of the outer and the inner bead compartments. Based on this segmentation information mean fluorescence intensities are quantified. The number of independent fluorescence signal intensities assigned to each bead is 4^{∗}2 = 8, considering four channels and two compartments.

### Bead Decoding

Based on fluorescence intensity levels of coding particles located in the inner compartments or the intensity level of the inner compartment itself, the bead decoding algorithm assigns each bead to one of the bead types specified by the underlying bead library.

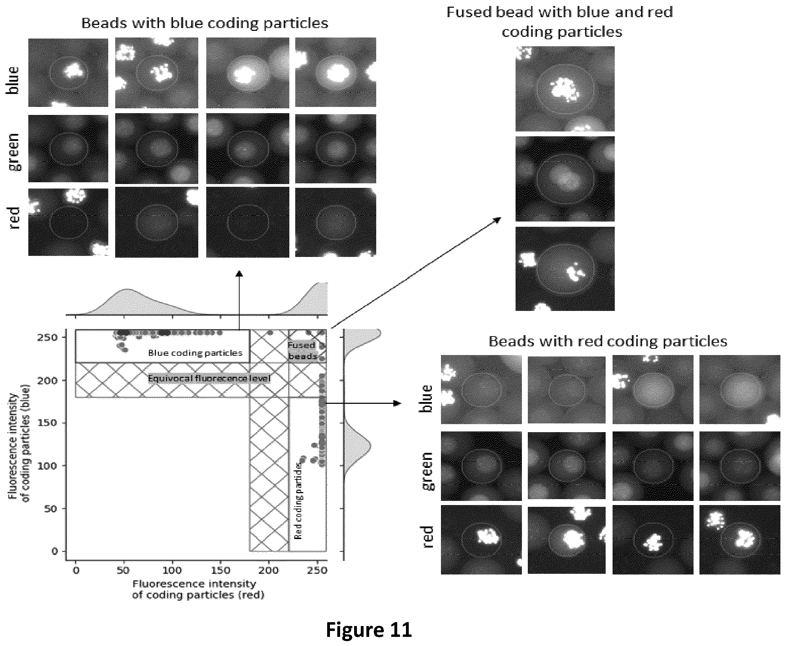

The embodiment illustrated in Figure 11 originates from an experiment with beads from two bead types labelled with either blue or red coding particles. Images were acquired at the end of PCR amplification. Beside coding particles in the blue and red channel, these channels also contain detection information for analytes. Thus, the outer bead compartment can be PCR positive or negative. Image acquisition conditions for each channel were adjusted to ensure that pixels that image coding particles with present dye are saturated. The absolute fluorescence level of coding particles if dye is not present results from the PCR outcome in the outer bead compartment. As a result, fluorescence levels in saturation or close to (e.g. 220 - 256) are classified as present coding particle. Beads with equivocal fluorescence levels (e.g. 180 - 220) are set to invalid. Middle and low intensities (e.g. < 180) are classified as absent coding particle. Fused beads with blue and red labels are set to invalid.

### Data Analysis

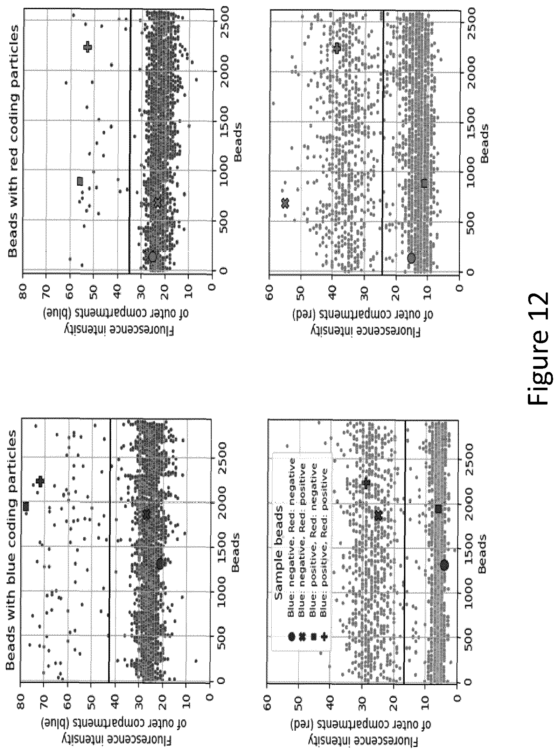

Final data analysis for analyte detection and quantification is based on fluorescence intensities measured in the outer bead compartments. Due to spatial separation of coding and detection information it is possible to obtain analyte specific information within the same fluorescence channel that is used for encoding the bead.Quantification can be performed either by digital or real-time PCR analysis approaches. Digital PCR analysis relies on fluorescence signals estimated from images at the end of PCR, while real-time analysis is based on fluorescence signals estimated from images during all cycles of the real-time PCR.Figure 12 shows fluorescence signal intensities of outer bead compartments in blue and red detection channel for embodiment in Figure 11. The four sample beads for each bead type cover the four possible combinations of PCR negative or positive results in blue and red channel respectively. The intensities of the eight sample beads marked in the plot of figure 11 are highlighted in intensity plots in Figure 12.

### Bead Fusing Control

Detection of fused beads is possible either by detection of existence of unknown code combinations (e.g. in case of single-color codes) or by detection of multiple inner compartments (see Figure 11).

### Example 3

### Binding and quantitation of bacterial gDNA with BLINK nanoreactor beads

Bacterial gDNA from methicillin-resistant Staphylococcus aureus (MRSA) strain ST8 USA300 with a genome length of 2.8 Mb was bound and quantified by digital PCR using BLINK nanoreactor beads. Cultured bacteria were mechanically lysed using ceramic beads and a Minilys Homogenizer instrument (Bertin Technologies) at agitation level 3 for 2 min. Lysate was diluted in PBS 1:2000 and stored at -20°C. Nanoreactor beads used consist of an agarose hydrogel scaffold (A9793, Sigma), a cross-linkedChitosan-HCl (Heppe Medical chitosan GmbH) capture matrix, a fluorescence coding label with Lumogen Red F 300 nanoparticles (Kremer Pigmente GmbH & Co. KG), a fluorescence label for segmentation (A9793-Cy3) and embedded 5µm polystyrene magnetic microparticles (Sigma) for retention.

### Binding & Washing

Nanoreactor beads, as produced in Example 1, are supplied as a freeze-dried pellet accommodated in 0.2ml processing tubes comprising a defined amount of 100µm beads (n=56000) for nucleic acid capturing and subsequent digital amplification reaction of a single sample. Nanoreactors are resuspended and prewashed in component 1 necessary to assure pre-conditioning of the capture matrix. Therefore, twice the volume of component 1 is added to the beads. The resulting bead slurry is placed into a magnetic rack to retain the beads, the supernatant is aspirated and discarded. Prewashing is repeated twice yielding nanoreactor Beads in an equilibrated state equipped for following nucleic acid binding.

### Component 1: Resuspension Buffer/ Pre-washing buffer

### 10mM MES (2-(N-morpholino)ethanesulfonic acid), pH 5.0

For binding DNA to nanoreactor beads, as produced in Example 1, 20µL of diluted lysate in 3 concentrations levels was pipetted to the 40µL bead bed containing 56000 beads followed by addition of 140µL binding buffer (Component 2). Binding mix were incubated at 25°C on an Eppendorf Thermomixer for 5 min at 1800 rpm. After capturing the supernatant was gently removed using a magnetic tube rack and a pipette where the beads were kept on the tube wall.

### Component 2: Binding Buffer

### 50mM Malonic Acid pH 3.5

A similar procedure was performed during washing when the beads were washed with 300µL washing buffer (Component 3) for 3 times to remove any unbound DNA and cell debris.

### Component 3: Washing Buffer

### Malonic Acid 12.5 mM (pH 3.5)

### Loading of detection reagents to the Nanoreactor Beads

Analyte-specific and generic reagents mecA target detection within the nanoreactors were supplied as a freeze-dried pellet that is resuspended with component 4. Sequences of primers and probe are targeting for mecA gene that encodes the protein PBP2A (penicillin-binding protein 2A) of methicillin-resistant Staphylococcus aureus (MRSA). Equal volumes of resulting component 5 and of analyte bound nanoreactor beads derived from the upstream sample preparation process are combined. The porous nanoreactor beads are allowed to take up the detection reagents by incubation at 25°C for 5min while shaking at 800rpm. Excess liquid is removed by retention of reagent loaded nanoreactor Beads by magnetic forces.

### Component 4 (Generic PCR Buffer)

### 2x RT-PCR Buffer: 40mM Tris HCl, 44mM KCl, 44mM NH₄Cl, 6mM MgCl₂

### Component 5 (Generic and Specific RT-PCR reagents)

2x RT-PCR Buffer: 40mM Tris HCl, 44mM KCl, 44mM NH₄Cl, 6mM MgCl2
0.4 U/µl Hot Start Taq DNA Polymerase (biotechrabbit GmbH)
0.8 mM dNTPs (biotechrabbit GmbH)
0.8µM sense primer (mecA_R: 5'- TGGCATGAGTAACGAAGAATATAA - 3') (SEQ ID NO:1) (Metabion International AG)
0.8µM antisense primer (mecA_R: 5'- GAGTTGAACCTGGTGAAGTTG -3') (SEQ ID NO:2) (Metabion International AG)
0.8µM TaqMan probes (mecA_P: 5'-Att0647N - AAAGAACCTCTGCTCAACAAGTTCCAGA - BHQ2-3') (SEQ ID NO:3) (Metabion International AG)
0.2% (w/v) low bioburden, protease free, for molecular biology BSA (Sigma)

### Phase transfer of loaded nanoreactor beads

Reagent-loaded nanoreactor beads are transferred and dispersed into a non-aqueous phase (component 6) to create individual nanoliter-sized reaction spaces. Thus, beads were emulsified by adding an excess of component 6 (100µl) to the Beads followed by a 2 times agitation step using a Minilys Homogenizer instrument (Bertin Technologies) at agitation level 2. The applied mechanical stress breaks attracting forces between the aqueous nanoreactor beads and creates surface tension forming a suspension/ emulsion. Both the hydrogel beads and excess aqueous phase are emulsified in the oil phase. The submicron-scaled droplets that are produced as a byproduct are eliminated by gentle aspiration with a pipette as microemulsion forms a separate layer below the emulsified nanoreactor beads. Repeated washing with the same emulsion/ amplification oil (component 6) removes essentially all undesirable liquid droplets. Additional 100µl of component 6 was added.

### Component 6 (Phase transfer oil)

- HFE-7500 fluorocarbon oil (3M Deutschland GmbH) supplemented with
- 2-5% (v/v) PicoSurf (SphereFluidics) OR 2-5% (v/v) FluorSurf (Emulseo)

### Digital PCR amplification, detection and gDNA quantitation in nanoreactor beads

Nanoreactor bead suspension was transferred into a reaction and detection chamber (RDC) made of polycarbonate and covered with a 250µm transmissive polycarbonate film to form an inner chamber height of app. 100 µm and allowing the beads to distribute as a mono layer within the chamber. An example of a suitable reaction and detection chamber is described in Loncarevic IF, et al., PLOS ONE 16(3): e0242529. https://doi.org/10.1371/journal.pone.0242529.

The RDC was then mounted on a high-performance Peltier element sitting on a test rig with the thin film of the chamber contacting the Peltier. PCR was performed using the following cycling conditions: 120s 95°C for initial denaturation, 45 cycles of 5sec 95°C, 15sec 61°C.

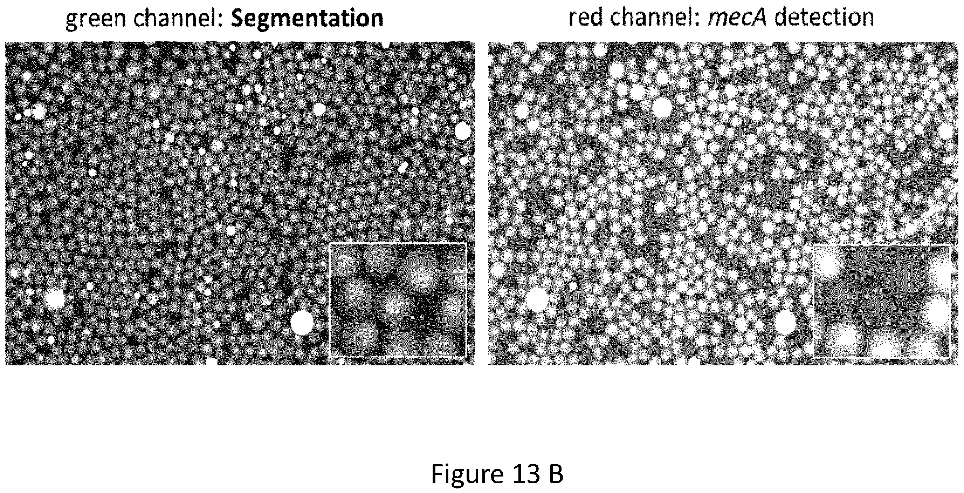

Upon completion of PCR protocol 2 images shown in figure 13B are acquired at each chamber position adding up to a total of 2×48 images. One channel is required for segmentation (green: Xexc 1 = 550nm), the second channel for mecA specific amplification signals (red: Xexc 2 = 635nm). Automated image acquisition is triggered by the BLINK toolbox software and is achieved by using the Fluorescence microscope (Zeiss AxioObserver) equipped with a 5x objective (field of view 4.416mm × 2.774mm) and a pE-4000 (CoolLED Ltd.) light source. The microscope was further equipped with three fluorescence filter sets (Cy5 ET, Cy3 ET, AHF Analysentechnik) and an automated x-y stage to which the thermocycler with the reaction chamber was mounted.

DNA input per target was quantified using by performing a Poison analysis based on the number of detected positive and negative beads per target. For each lysate concentration level a diameter normalized lambda value was calculated. Lambda values were multiplied with initial number of beads used for binding to calculate gDNA copies output (cp output).

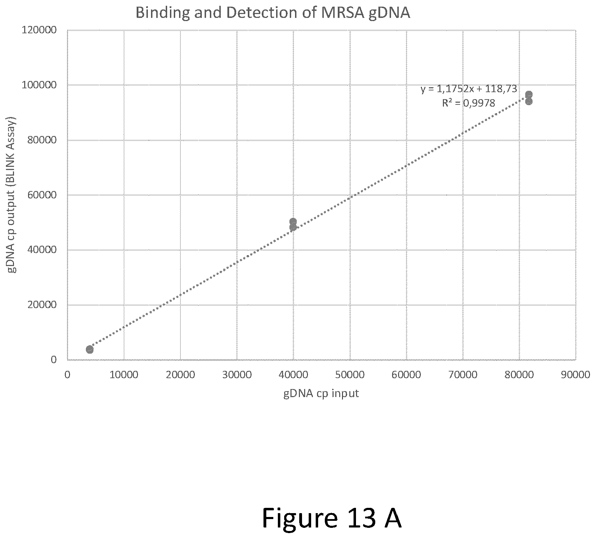

Nominal input was calculated by qPCR using a standard calibration curve shown in figure 13A.

### Example 4

### Nucleic acid extraction with nanoreactor beads from crude samples

In the following embodiment, a recombinant Accuplex^{™} (SeraCare Life Sciences, Inc.) HCV high titer control material (Lot No. 10321228) spiked EDTA plasma sample is encapsulated in a monodispersed emulsion using nanoreactor beads in accordance with the present invention which integrates RNA extraction and detection into a single workflow. The Accuplex^{™} control material is based on the Sindbis virus resembling the complexity of a virus found in true patient samples and contains HCV specific RNA sequences. Nanoreactor beads used consist of an agarose hydrogel scaffold (A9793, Sigma), a cross-linkedChitosan-HCl (Heppe Medical chitosan GmbH) capture matrix, a fluorescence coding label with Lumogen Red F 300 nanoparticles (Kremer Pigmente GmbH & Co. KG), a fluorescence label for segmentation (A9793-Cy3) and embedded 5µm polystyrene magnetic microparticles (Sigma) for retention. They were produced essentially as described in Example 1.

### Preparation of nanoreactors

The nanoreactor beads are supplied as a freeze-dried pellet accommodated in 0.2ml processing tubes comprising a defined amount of 100µm beads (n=53000) for nucleic acid purification and subsequent digital amplification reaction of a single sample. The nanoreactors are resuspended and prewashed in component 1 necessary to assure pre-conditioning of the capture matrix. Therefore, twice the volume of component 1 is added to the beads. The resulting bead slurry is placed into a magnetic rack to retain the beads, the supernatant is aspirated and discarded. Prewashing is repeated twice yielding nanoreactor beads in an equilibrated state equipped for following nucleic acid binding.

### Component 1: Resuspension Buffer/ Wash Buffer

- 10mM MES (2-(N-morpholino)ethanesulfonic acid), pH 6.5

### Bead-based RNA extraction

50µl plasma prepared by centrifugation of HCV negative EDTA whole blood (supplied by Universitätsklinikum Jena) is spiked with diluted Accuplex^{™} HCV control material and is directly applied to the equilibrated nanoreactor beads. The original viral titer was 2.69 ×10⁸ cp/ml according to manufacturer's information. Control material was diluted in nuclease-free water to a final viral titer of 2.69 ×10⁷ cp/ml of which 1µl was spiked corresponding to an analyte input size of 26900 viral particles. Chaotropic agent-containing component 2 is added to the processing tube at a volume of 133µl followed by incubation at 30°C while shaking at 1800rpm for 5min for efficient sample homogenization, lysis of viral particles and stabilization of released and fragile RNA. Binding of RNA is accomplished by addition of a Binding Buffer (component 3) to a final binding volume of 300µl and repeated incubation at 25°C while shaking at 2000rpm for an additional duration of 5min. Alternatively, lysis and binding can be combined into a single process step (10 min incubation at 25°C and agitation at 1800 rpm) using the same mixture with identical volumes of component 2, component 3, nanoreactor beads and EDTA Plasma. The magnetized microparticles/nanoparticles contained within the nanoreactor beads allow for transfer of the nanoreactors through the different sample preparation solutions including a wash buffer (component 1). Thus, the processing tubes are moved to a magnetic rack to retain the beads. The binding supernatant is removed and the nanoreactors are resuspended in 300µl of component 1. Repeated washing (5x) efficiently removes essentially most potential inhibitors that may interfere with downstream enzymatic reactions making the bead-based sample processing compatible with the subsequent digital amplification reactions which are both performed within the same compartments.

### Component 2: Lysis Buffer

- 100mM Acetate Buffer solution, pH4 (Alfa Aesar)
- 3M Guanidine Sulfate (Sigma)
- 20mM Ethylenediaminetetraacetic acid (Acros Organics)
- 10% (v/v) Tergitol (Sigma)

### Component 3: Binding Buffer

- 1M MES (2-(N-morpholino)ethanesulfonic acid), pH 6.5

### Loading of detection reagents to the nanoreactor beads

The analyte-specific and generic reagents for HCV detection within the nanoreactors were supplied as a freeze-dried pellet that is resuspended with component 4. Equal volumes of resulting component 5 and of analyte bound nanoreactor beads derived from the upstream sample preparation process are combined. The porous nanoreactors are allowed to take up the detection reagents by incubation at 30°C for 5min while shaking at 1000rpm. Excess liquid is removed by retention of reagent loaded nanoreactor beads by magnetic forces.

### Component 4 consists of the following final concentrations

- 2x RT-PCR Buffer: 40mM Tris HCl, 44mM KCl, 44mM NH₄Cl, 6mM MgCl₂

### Component 5:

- 2x RT-PCR Buffer: 40mM Tris HCl, 44mM KCl, 44mM NH₄Cl, 6mM MgCl2
- 0.4 U/µl Hot Start Taq DNA Polymerase (biotechrabbit GmbH)
- 0.8 mM dNTPs (biotechrabbit GmbH)
- 0.8µM sense primer (HCV_Brun_F: 5'-GTGGTCTGCGGAACCGGTGA-3') (SEQ ID NO:4) (Metabion International AG)
- 0.8µM antisense primer (HCV_Brun_R: 5'-CGCAAGCACCCTATCAGGCAGT-3') (SEQ ID NO:5) (Metabion International AG)
- 0.8µM TaqMan probes (HCV_Brun_Pi: 5'-Att0488-CCGAGTAGCGTTGGGTTGCGAAAGG-BHQ1-3') (SEQ ID NO:6) (Metabion International AG)
- 0.2% (w/v) low bioburden, protease free, for molecular biology BSA (Sigma)

### Phase transfer of loaded nanoreactors

Reagent-loaded nanoreactors are transferred and dispersed into a non-aqueous phase (component 6) to create individual nanoliter-sized reaction spaces. Thus, beads were emulsified by adding an excess of component 6 (100µl) to the Beads followed by a 2 times agitation step using a Minilys Homogenizer instrument (Bertin Technologies) at agitation level 2. The applied mechanical stress breaks attracting forces between the aqueous Beads and creates surface tension forming a suspension/ emulsion. Both the hydrogel beads and excess aqueous phase are emulsified in the oil phase. The submicron-scaled droplets that are produced as a byproduct are eliminated by gentle aspiration with a pipette as microemulsion forms a separate layer below the emulsified nanoreactor beads. Repeated washing with the same emulsion/ amplification oil (component 6) removes essentially all undesirable liquid droplets. Additional 100µl of component 6 was added.

### Component 6: Phase transfer & Signal amplification oil

- HFE-7500 fluorocarbon oil (3M Deutschland GmbH) supplemented with
- 2-5% (v/v) PicoSurf (Dolomite Microfluidics) OR 2-5% (v/v) FluoSurf (Emulseo)

### Digital RT-PCR amplification in nanoreactors

Nanoreactor bead suspension was transferred into a reaction and detection chamber (RDC) made of polycarbonate and covered with a 250µm transmissive polycarbonate film to form an inner chamber height of app. 100 µm and allowing the beads to distribute as a monolayer within the chamber (1) forming a suspension array. The RDC was mounted on a high-performance Peltier element with the thin film of the chamber contacting the Peltier. nanoreactors are subjected to rapid temperature cycling and an established chamber-specific PCR control mode. The thermal conditions applied were: Reverse transcription for 10min at 50°C, Initial denaturation for 30s at 95°C followed by 30-45 cycles of a two-step PCR consisting of Denaturation at 95°C for 5s and Annealing/Elongation at 62°C for 10s. Due to their sol-gel switching capability, the suspension becomes an emulsion with individual liquid nanoreactor compartments.

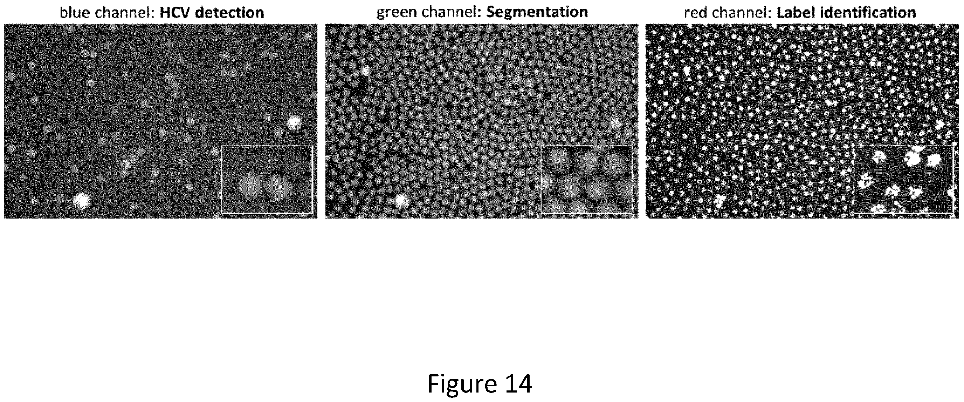

Upon completion of the thermal protocol 3 images, as shown in Figure 14, are acquired at each chamber position adding up to a total of 2×48 images. One channel is required for label identification (red: Xexc 1 = 650nm), the second channel for HCV-specific amplification signals (blue: Xexc 2 = 470nm) and the third channel for segmentation (green: Xexc 3 = 550nm). Automated image acquisition is triggered by the BLINK toolbox software and is achieved by using the Fluorescence microscope (Zeiss AxioObserver) equipped with a 5x objective (field of view 4.416mm × 2.774mm) and a pE-4000 (CoolLED Ltd.) light source. The microscope was further equipped with three fluorescence filter sets (Cy5 ET, Cy3 ET, FITC/FAM HC, AHF Analysentechnik) and an automated x-y stage to which the thermocycler with the reaction chamber was mounted.

### Bead Segmentation and Endpoint Analysis

All images acquired are subjected to an automated multifaceted image processing algorithm developed by BLINK to detect and segment individual nanoreactor beads. Features including fluorescence signals in each channel, position, diameter/volume, etc. of all segmented nanoreactors are subsequently collected. Experimental data is processed by the open-source software Jupyter.

Droplet digital PCR data for Accuplex HCV is viewed in a 1-D or 2-D plot. The software clusters the negative and positive fractions for each target and fits the fraction of positive events to a Poisson algorithm to determine the output copy number expressed as a λ-value (normalized to 100µm Bead diameter). λ-values were multiplied with the input number of nanoreactor beads to determine the output HCV copy number, respectively. A mean of 6697 copies with a standard deviation of SD=258 copies (n=2) were recovered from the integrated sample preparation process and digital detection.

### Example 5

### Performing amplification and real time detection using primers that have been bound to the first polymer

Biotinylated agarose as described in WO 2021/122563A1 has been used for generating beads comprising biotinylated agarose as the first polymer according to the protocol in example 1. Analyte specific beads are generated by binding target specific primers and probes to precursor beads with defined fluorescent codes. All bead handling and wash steps are performed in a single laboratory tube (1.5 ml) with a magnetic rack. Precursor beads (25% volume bead bed in 100 mM Tris-buffer, pH 8.9) are first derivatized with Streptavidin (0.5 mg/ml in 100 mM Tris-buffer, pH 8.9) at room temperature under constant shaking at 800 rpm for 30 min on a thermomixer. Excess Streptavidin is removed by 3-times washing in water with 10fold volume. By the addition of 5'biotin to the primers and 3'biotin to the probes, the bead-bound PCR reaction shows similar performance compared to PCR using unbound oligonucleotides. Primers and probes are immobilized to the beads in 100 mM tris buffer, pH 8.9, at room temperature under constant shaking at 800 rpm for 30 min on a thermomixer. After incubation, unbound oligonucleotides are removed with 5-fold volume of water. The biotin/streptavidin bond remains stable under the acidic conditions of the subsequent capture of genomic DNA (50 mM malonic acid, pH 2.5) to the cross-linked chitosan polymer of the beads (15 min at room temperature und shaking at 800rpm on a thermomixer). After DNA capture, the beads are washed three times in 5-fold bead volume with malonic acid (12.5 mM, pH 5.0), subsequently 2x concentrated PCR mix without oligonucleotides is loaded onto the beads. Thereafter 100 µl of Picosurf oil (Spherefluidics) is given to the suspension. The tube is closed and mounted on a bead beating device (Omnilyse, Bertin) and shaken at 50% speed three times for 5 sec. Thereafter the bead suspension is transferred to a suitable detection chamber for PCR incubation and image acquisition.

### Detection of genomic targets using Taqman-PCR probes in realtime PCR

Beads with Cy3-stained agarose providing for background for image segmentation are preloaded with desthio-biotinylated primers and Cy5/desthiobiotin labeled probe for the detection of gapA species marker of *Staphylococcus aureus* (0.5 µM forward primer, 0.5 µM reverse primer, 0.4 µM probe). As a negative control a population of beads labelled with FAM and without any bound primers and probes is used.

Capture of genomic DNA isolate of *S.aureus* (input concentration set to 100 genomic copies per bead) is performed by re-suspending the beads in 100µL of binding buffer (100 mM malonic acid, pH 2.5) und mixing the bead suspension with the same amount of sample. Following an incubation for 15 min at room temperature und shaking at 800rpm on a thermomixer, the beads are washed three times in 5-fold bead volume with malonic acid (12.5 mM, pH 5.0). Subsequently 2x PCR mastermix is loaded on the beads, resulting in the following PCR buffer composition on the beads:
100 mM Tris/HCl pH 8.9
22 mM ammonium chloride
22 mM potassium chloride
3 mM MgCl2
0.35 mM dNTPs
0.1% BSA
0.2 Units DNA Polymerase

Primers/probe sequences used for this analysis are:

| Oligo | Name | 5'- tag | 3'-tag | sequence |
|---|---|---|---|---|
| Primer forward | gapA for | Desthiobiotin | - | cgtCAAtTagTtAAAgttGCaG (SEQ ID NO:7) |
| Primer reverse | gapA rev | Desthiobiotin | - | gctAagTatgcTAAtGTACGaAC (SEQ ID NO:8) |
| Probe | Cy5 gapA | Desthiobiotin | Cy5 | TGGTAtGAtAAcGAAATGTCaTAtACTgCaC (SEQ ID NO:9) |

After re-suspension in oil, the amplification is performed (2 min initial denaturation, 42 cycles of 10 sec denaturation at 95°C, followed by 15 sec at 58°C for annealing/elongation). The PCR reaction is monitored in real time by fluorescence imaging on a Zeiss Axio Observer Fluorescence microscopy at every cycle during the annealing/elongation phase.

The image analysis algorithm segments beads on images by recognition of their bright disk-shaped object contours in the green channel, leading to Cy3 generated signals. The resulting segmentation information allows the estimation of mean fluorescence levels for all beads across all cycles (1 to 42) and in all relevant channels (red/Cy5 for PCR probe signal and blue/Fam for the coding of control beads). All beads are assigned to a bead type using the signal intensity level measured in the blue channel (FAM) in the first cycle. Analyte specific beads are characterized by low signal intensity while beads of the negative control group have a high signal value.

In the field of view shown in the upper left panel of figure 15, the total number of valid positive beads is 618 and the total number of valid control beads is 33. The real-time analysis algorithm fits a nonlinear function to the fluorescence signal course of each single bead, as shown in the lower left panely of figure 15. The fitted nonlinear model combines a sigmoid and a linear function where the sigmoid component reveals amplification kinetics, and the linear component represents the baseline of the signal. The cycle threshold value (Ct) is calculated from the intersection of tangent in the definition value of sigmoid function with maximum second derivation and baseline, as shown in the right panel of figure 15.

More specifically, figure 9 shows the monitoring of bead digital PCR reaction with detection of *S. aureus* genomic DNA

Figure 9 upper left panel: shows the field of view for the Cy5 channel after amplification (PCR signal, bright and dark beads are clearly visible). The Cy3 image taken before PCR reveals the analyte specific beads, the FAM image shows the control bead. The corresponding signals in the Cy5 image show that all analyte specific beads show positive PCR signals whereas the control bead remains dark. Because the target concentration is set at a very high level (app 100 cp/bead) in this example very high all analyte specific beads show a bright Cy5 signal. The graph on the lower left panel shows the real-time curves for Cy5 fluorescence for the individual beads shown in the insets and the average intensity for the PCR postive and negative beads. Three beads belong to the positive bead type and exhibit strong amplification kinetics with Ct values of app. 21.6 and lift ratios of app. 2.5. The density distribution of Ct values estimated from all 618 valid beads of positive bead type is illustrated on the right side of figure 15. The fourth bead is a control bead without increase in fluorescence intensity. Alternatively to the analysis of real-time curves of all beads separately and the subsequent summary of Ct values, the method also allows the calculation of a single mean fluorescence intensity per bead type.

As shown in Figure 15 (left side) for the present embodiment, there are two mean real-time curves, one for the positive bead type and one for the negative control bead type.

The features of the present invention disclosed in the specification, the claims, and/or in the accompanying drawings may, both separately and in any combination thereof, be material for realizing the invention in various forms thereof.

## Claims

1. A microsphere comprising a first material and a second material, wherein the first material is capable of forming a porous hydrogel when exposed to or comprising an aqueous solution, and has a melting temperature in the range of from 40°c to 90°C; and wherein the second material forms a network within said porous hydrogel, or wherein said second material is attached to said first material and both materials together form a network within said porous hydrogel; wherein said second material has a pKa-value and is capable of precipitating in an aqueous environment at a pH ≥ said pKa-value; wherein said network is capable of contracting when exposed to a temperature ≥ said melting temperature of said first material; said microsphere further comprising at least one of a) a fluorescent label and b) magnetic particles.

2. The microsphere according to claim 1, wherein said first material is a first polymer, and wherein said second material is a second polymer, an oligomer or a monomer; wherein
- if said second material is a second polymer, said second polymer is a polymer cross-linked with itself or is a polymer cross-linked with said first polymer;
- if said second material is an oligomer or a monomer, said oligomer or said monomer is attached to said first polymer.

3. The microsphere according to any of claims 1 - 2, wherein said second material, preferably said second polymer or said oligomer or said monomer, is furthermore capable of binding nucleic acids at a pH < said pKa-value, wherein, preferably, said pKa-value of said second material, preferably of said second polymer or of said oligomer or of said monomer, is > 6.

4. The microsphere according to any of claims 1 - 3, wherein said fluorescent label is selected from a) fluorescent particles having a size in the range of from 50 nm to 10 µm, preferably 100nm to 5 µm, more preferably 1 µm to 5 µm, and even more preferably 1 µm to 3 µm; b) non-particulate fluorescent dyes attached to said first and/or second material, preferably to said first and/or second polymer/said oligomer/said monomer; and c) combinations of a) and b).

5. The microsphere according to any of claims 1 - 4, wherein said magnetic particles have a size in the range of from 50 nm to 10 µm, preferably 100 nm to 5 µm, more preferably 1 µm to 5 µm, and even more preferably 1 µm to 3 µm.

6. The microsphere according to any of the foregoing claims, wherein said first material, preferably said first polymer, comprises a binding member allowing the reversible and pH-independent immobilization of one or several amplification primers to said first material, preferably to said first polymer, wherein, preferably, for such reversible and pH-independent immobilization, said binding member specifically interacts with a binding entity attached to said one or several amplification primers; wherein, more preferably, said microsphere further comprises one or several amplification primers having a binding entity attached and being immobilized to said first material, preferably said first polymer, via said specific interaction between said binding member on said first material, preferably said first polymer, and said binding entity on said one or several amplification primers; wherein, even more preferably, said binding member is selected from avidin, streptavidin and derivatives thereof, and said binding entity is selected from desthiobiotin, iminobiotin, biotin having a cleavable spacer arm, selenobiotin, oxybiotin, homobiotin, norbiotin, diaminobiotin, biotin sulfoxide, biotin sulfone, epibiotin, 5-hydroxybiotin, 2-thiobiotin, azabiotin, carbobiotin, methylated derivatives of biotin, and ketone biotin; or vice versa.

7. The microsphere according to any of the foregoing claims, wherein said first material is a first polymer and is selected from agarose, gelatin, hyaluronan, elastin, elastin-like polypeptides, thermoresponsive polymers having an upper critical solution temperature (UCST), e.g. selected from poly(N-acryloyl glycinamide) (PNAGA), poly(allylamine)-co-poly(allylurea) and its derivatives, poly(methacrylamide), poly(N-acryloylaspargineamide), poly(N-methacryloylglutamineamide), poly(acrylamide)-co-(acrylonitrile). poly(sulfobetaine)s, poly(phosphorylcholine)s; and other polymers capable of forming a porous hydrogel when exposed to or comprising an aqueous solution, and having a melting temperature in the range of from 40°c to 90°C ; and said second material is either a second polymer selected from chitosan and derivatives thereof, gelatin and derivatives thereof, methylcellulose, poly(N-isopropylacrylamide) (pNIPAM), poly(ethyleneimine), poly(2-dimethyl(aminoethyl)methacrylate), poly(lysine), poly(histidine), poly(arginine) and polymer backbones having basic amino acids attached or incorporated as part of such backbone; or said second material is an oligomer, preferably selected from oligopeptides, said oligopeptides comprising or consisting of basic amino acid, such as histidine, lysine, and arginine; or said second material is a monomer selected from basic amino acids, such as histidine, lysine, and arginine;
with the proviso that when said first and said second material are a first and second polymer respectively, said polymers are different;
wherein, preferably, said first material is a first polymer that is non-cross-linked, and said second material is a second polymer that is cross-linked; or, equally preferably, said first material is a first polymer and said second material is a second polymer cross-linked with said first polymer; wherein, more preferably, said first polymer is agarose, and said second polymer is cross-linked chitosan.

8. The microsphere according to any of claims 1 - 7, comprising an aqueous solution, such that said first material, preferably said first polymer is in the form of a porous hydrogel.

9. The microsphere according to claim 8, further comprising nucleic acid(s) which is(are) not an oligonucleotide primer or oligonucleotide primers, and which is(are)
- bound to said second material, preferably to said second polymer, if said aqueous solution has a pH< said pKa-value of said second material, preferably said second polymer, and
- still retained within the microsphere by or in said porous hydrogel of said first material, preferably said first polymer, if said aqueous solution has a pH ≥ said pKa-value of said second material, preferably said second polymer, but not necessarily bound or not bound anymore to said second material, preferably said second polymer.

10. The microsphere according to any of claims 8 - 9, wherein said aqueous solution is either:
a) a first composition for washing and/or facilitating the binding of nucleic acids to said second material, e.g. said second polymer, said first composition having a pH-value < said pKa-value of said second material, e.g. said second polymer or, more preferably, said first composition comprising a buffer solution buffering in a pH-range < pKa-value of said second material, e.g. said second polymer; or
b) a second composition for facilitating and performing a nucleic acid amplification, said second composition comprising a buffer buffering in a pH-range suitable for performing a nucleic acid amplification, mono-nucleoside-triphosphates, an amplification enzyme, such as a suitable nucleic acid polymerase, e.g. Taq polymerase, optionally one or more amplification primers or one or more pairs of amplification primers if not already immobilized to said first material, preferably to said first polymer; and optionally a nucleic acid dye for the detection of an amplification product, such as an amplified nucleic acid; and/or, optionally, comprising respective molecular probes, e.g. TaqMan Probes, or molecular beacons;; wherein said pH-range suitable for performing a nucleic acid amplification is > said pKa-value of said second material, e.g. said second polymer.

11. The microsphere according to any of claims 1 - 10, wherein said second material, preferably said second polymer is in a non-precipitated form.

12. The microsphere according to any of claims 1 - 10, wherein said second material, preferably said second polymer is in a precipitated form.

13. A liquid phase that is immiscible with water and aqueous solutions, for example an oil phase, said liquid phase comprising a plurality of microspheres according to claim 11, or comprising a plurality of microspheres according to claim 12.

14. A method for capturing and/or enriching a nucleic acid or nucleic acids from a sample, said method comprising:
EITHER
a) Providing a plurality of microspheres as defined in any of claims 1 - 8 in a first composition for facilitating the binding of nucleic acids to said second material, preferably said second polymer, or exposing said plurality of microspheres to such first composition; said first composition having a pH-value < said pKa-value of said second material, e.g. said second polymer or, preferably, said first composition comprising a buffer solution buffering in a pH-range < pKa-value of said second material, e.g. said second polymer; thus allowing said plurality of microspheres to equilibrate with and incorporate said first composition; wherein preferably, said pKa-value of said second material, e.g. of said second polymer is > 6; and
b) Exposing said plurality of microspheres from step a) to a sample containing, or suspected of containing, nucleic acid(s), thus allowing said nucleic acid(s), if present, to bind to said second material, e.g. said second polymer, said sample, preferably, also comprising a buffer solution buffering in a pH-range < pKa-value of said second material, e.g. said second polymer, thereby capturing nucleic acid(s), if present in said sample;
OR
a') exposing a plurality of microspheres, as defined in any of claims 1 - 8 to a sample containing, or suspected of containing, nucleic acid(s), thus allowing said nucleic acid(s), if present, to bind to said second material, e.g. said second polymer, said sample having a pH < pKa-value of said second material, e.g. said second polymer, wherein, preferably, said sample comprises a buffer solution buffering in a pH-range < pKa-value of said second material, e.g. said second polymer; thereby capturing nucleic acid(s), if present in said sample, as a result of such exposure.

15. A method of amplifying a nucleic acid from a sample, said method comprising performing the method of claim 14; and thereafter:
EITHER (OPTION A):
c) exposing said plurality of microspheres from step b) or step a') to a second composition for facilitating and performing a nucleic acid amplification which comprises a second buffer solution buffering in a pH-range suitable for performing a nucleic acid amplification, mono-nucleoside-triphosphates, an amplification enzyme, such as a suitable nucleic acid polymerase, e.g. Taq polymerase, and, if said microspheres do not already comprise one or several amplification primers immobilized to said first material, preferably to said first polymer, via a specific interaction between a binding member on said first material, preferably said first polymer, and a binding entity on said one or several amplification primers, further comprising one or more amplification primers or one or more pairs of amplification primers; thus allowing said plurality of microspheres to equilibrate with and incorporate said second composition for facilitating and performing a nucleic acid amplification; said second composition, optionally, further comprising a nucleic acid dye for the detection of an amplification product, such as an amplified nucleic acid, and/or respective molecular probes, e.g. TaqMan Probes, or molecular beacons; wherein preferably, said pH-range suitable for performing a nucleic acid amplification is > said pKa-value of said second material, e.g. said second polymer;
d) subjecting said plurality of microspheres of step c) to an amplification protocol within said composition for facilitating and performing a nucleic acid amplification, such protocol involving or being preceded or followed by at least one step in which said suspension is heated to a temperature > melting temperature of said first material, preferably of said first polymer, such protocol, preferably, involving repeatedly and cyclically increasing and lowering the temperature to which said suspension of said plurality of microspheres is exposed, to levels allowing the amplification of nucleic acid(s), if present in said microspheres, that match the sequence(s) of said one or more amplification primers or one or more pairs of amplification primers;
OR (OPTION B):
e) exposing said plurality of microspheres from step b) or step a') to a second composition for facilitating and performing a nucleic acid amplification which comprises a second buffer solution buffering in a pH-range suitable for performing a nucleic acid amplification, mono-nucleoside-triphosphates, an amplification enzyme, such as a suitable nucleic acid polymerase, e.g. Taq polymerase, and, if said microspheres do not already comprise one or several amplification primers immobilized to said first material, preferably to said first polymer, via a specific interaction between a binding member on said first material, preferably said first polymer, and a binding entity on said one or several amplification primers, further comprising one or more amplification primers or one or more pairs of amplification primers; thus allowing said plurality of microspheres to equilibrate with and incorporate said second composition for facilitating and performing a nucleic acid amplification; said second composition, optionally, further comprising a nucleic acid dye for the detection of an amplification product, such as an amplified nucleic acid, and/or respective molecular probes, e.g. TaqMan Probes, or molecular beacons; wherein preferably, said pH-range suitable for performing a nucleic acid amplification is > said pKa-value of said second material, e.g. said second polymer;
f) transferring said plurality of microspheres from step e) to a liquid phase that is immiscible with water and aqueous solutions, for example an oil phase, thereby generating a suspension of said plurality of microspheres that are isolated from each other by said liquid water-immiscible phase, e.g. oil phase; and
g) subjecting said plurality of microspheres of step f) to an amplification protocol, such protocol involving or being preceded or followed by at least one step in which said suspension is heated to a temperature > melting temperature of said first material, preferably said first polymer, such protocol, preferably, involving repeatedly and cyclically increasing and lowering the temperature to which said suspension of said plurality of microspheres is exposed, to levels allowing the amplification of nucleic acid(s), if present in said microspheres, that match the sequence(s) of said one or more amplification primers or one or more pairs of amplification primers, wherein, as a result of heating to a temperature > melting temperature of said first material, preferably said first polymer, the second material, preferably said second polymer, in said microspheres contracts, thereby generating a contracted core within each microsphere, wherein the fluorescent label and/or the magnetic particles, if present or comprised in the respective microsphere, accumulate within said contracted core, thus facilitating the detection and/or further manipulation of the respective microsphere, wherein, preferably, i) said fluorescent label and/or ii) said magnetic particles, accumulated within said contracted cores of said microspheres, are used to locate and/or identify individual microspheres or a plurality of microspheres.

16. The method according to claim 15, further comprising the step(s):
h) Detecting amplified nucleic acid(s), if present,
EITHER in said composition for facilitating and performing a nucleic acid amplification (OPTION A),
OR in said microspheres (OPTION B);
wherein said amplification protocol results in the generation of an optically detectable signal which optically detectable signal is generated
EITHER in said composition for facilitating and performing a nucleic acid amplification (OPTION A),
OR in a space surrounding said contracted core of the respective microsphere (OPTION B);
and wherein detection of said optically detectable signal indicates the presence of amplified nucleic acid.

17. The method according to any of claims 15 - 16, wherein individual microspheres or a plurality of microspheres are located and/or identified using i) said fluorescent label and/or ii) said magnetic particles, accumulated within said contracted cores of said microspheres.

18. The method according to any of claims 15 - 17, wherein fused microspheres are distinguished from non-fused microspheres using i) said fluorescent label and/or ii) said magnetic particles, accumulated within said contracted cores of said microspheres; and/or iii) the presence of more than one contracted core within a fused microsphere; and/or iv) the presence of only one contracted core within a non-fused microsphere.

19. Use of a plurality of microspheres as defined in any of claims 1 - 12 in an assay for the detection of multiple nucleic acid analytes, said use comprising:
performing the method according to any of claims 15 - 18, and wherein said use involves different pluralities of microspheres, each plurality of microspheres comprising a different fluorescent label and being specific for the detection of a particular nucleic acid analyte by virtue of comprising a specific amplification primer for the amplification of said particular nucleic acid analyte or a specific pair of such amplification primers, wherein said different pluralities of microspheres can be detectably distinguished from one another by means of said fluorescent label, and wherein each plurality of microspheres is specific for a different nucleic acid analyte.

20. Use of a plurality of microspheres as defined in any of claims 1 - 12 in an assay for the detection of a single nucleic acid analyte in multiple samples, said use comprising: performing the method according to any of claims 15 - 18, and wherein said use involves different pluralities of microspheres, each plurality of microspheres comprising a different fluorescent label and further comprising an amplification primer for the amplification of said single nucleic acid analyte or a pair of such amplification primers, each plurality of microspheres being specific for a particular sample by virtue of being separately exposed in step b) or a') to such particular sample, wherein said different pluralities of microspheres can be detectably distinguished from one another by means of said fluorescent label, and wherein each plurality of microspheres is specific for the same nucleic acid analyte, and is specific for a different sample.
